# EUROPÄISCHE PATENTANMELDUNG

(11) **EP 3 141 900 A1**
(43) Veröffentlichungstag der Anmeldung: **15.03.2017**
(21) Anmeldenummer: 16151334.6
(22) Anmeldetag: 14.01.2016
(51) Int. Cl.: G01N 33/49, G01N 11/16, C12Q 1/56

(54) **KARTUSCHE FÜR EINE KOAGULATIONSMESSUNG**

(30) Priorität: 08.09.2015 DE 202015104762 U
(71) Anmelder: Schulz, Jürgen, 21266 Jesteburg (DE); Taborski, Uwe, 61231 Bad Nauheim (DE)
(72) Erfinder: Schulz, Jürgen, 21266 Jesteburg (DE)
(74) Vertreter: Albrecht, Ralf

(57) **Zusammenfassung**

Kartusche zum Einsetzen in eine Vorrichtung zur Koagulationsmessung, mit einem Gehäuse, in dem ein Probenraum zum Aufnehmen eines Probenfluids ausgebildet ist, und einem in dem Gehäuse vorgesehen Schwingelement, das einen mit dem Gehäuse verbundenen und von diesem in den Probenraum vorstehenden länglichen, in wenigstens einer Schwingrichtung elastisch biegbaren Schwingabschnitt und einen an dem freien Ende des Schwingabschnitts angeordneten und wenigstens eine Haftfläche für das Probenfluid definierenden Probenkontaktabschnitt aufweist, wobei der Schwingabschnitt stabförmig mit einer insbesondere rechteckigen oder kreisförmigen Querschnittskontur ausgebildet ist, der quer zu der Längsrichtung des Schwingabschnitts und quer zu der wenigstens einen Schwingrichtung des Schwingabschnitts eine Breite aufweist, die zu einer maximalen Breite des Probenkontaktabschnitts in einem Verhältnis von höchstens 0,2, vorteilhaft höchstens 0,1 steht.

Ferner betrifft die vorliegende Erfindung eine Vorrichtung zur Koagulationsmessung mittels einer derartigen Kartusche.

## Beschreibung

Die vorliegende Erfindung betrifft eine Kartusche zum Einsetzen in eine Vorrichtung zur Koagulationsmessung, mit einem Gehäuse, in dem ein Probenraum zum Aufnehmen eines Probenfluids ausgebildet ist, und einem in dem Gehäuse vorgesehenen Schwingelement, das einen mit dem Gehäuse verbundenen und von diesem in den Probenraum vorstehenden länglichen, in wenigstens einer Schwingrichtung elastisch biegbaren Schwingabschnitt und einen an dem freien Ende des Schwingabschnitts angeordneten und wenigstens eine Haftfläche für das Probenfluid definierenden Probenkontaktabschnitt aufweist. Ferner betrifft die vorliegende Erfindung eine Vorrichtung zur Koagulationsmessung mittels einer erfindungsgemäßen Kartusche.

Vorrichtungen zur Koagulationsmessung (Gerinnungsmessung) sind im Stand der Technik in unterschiedlichen Ausgestaltungen bekannt und dienen dazu, den Gerinnungsstatus eines Patienten zu bestimmen und/oder das Maß einer therapeutisch gewünschten Gerinnungshemmung zu überwachen. Typischerweise werden derartige Vorrichtungen zur Koagulationsmessung verwendet, um die Blutgerinnung eines Patienten während medizinischer Operationen zu beurteilen. Während eines chirurgischen Eingriffs werden nur etwa 10 % der bei der Operation eröffneten Blutgefäße eines Patienten durch den Operateur verschlossen. Die verbleibenden 90 % der Blutgefäße müssen sich durch Blutgerinnung selbsttätig verschließen. Dementsprechend gelingt eine medizinische Operation nur bei einer ausreichenden Gerinnungsfähigkeit des Blutes des Patienten, die sicherzustellen und bei Bedarf durch eine entsprechende Medikamentengabe herbeizuführen ist.

Die Blutgerinnung kann sich im Kontakt des Blutes mit Fremdoberflächen (exogene Blutgerinnung) oder bei einer Verletzung des Blutgefäßsystems im Kontakt des Blutes mit Gewebethromboplastin (endogene Blutgerinnung) vollziehen. In einer frühen Phase des mehrstufigen Gerinnungsprozesses (Gerinnungskaskade) werden die Blutplättchen (Thrombozyten) aktiv, wodurch thrombozytäre Rezeptoren exprimiert und thrombozytäre Inhaltsstoffe freigesetzt werden, die den weiteren Verlauf der Blutgerinnung beeinflussen oder beschleunigen können. Am Ende der Gerinnungskaskade bildet sich aus dem im Blut gelösten Fibrinogen ein Fibringerinnsel. Durch entsprechende Aktivierung kann ein im Verlauf der Blutgerinnung bereits entstandenes Fibringerinnsel wieder in Lösung gebracht werden (Fibrinolyse).

Frühe Verfahren zur postoperativen Messung der Blutgerinnung beschränkten sich darauf, die für die beschriebene Gerinnungskaskade erforderlichen Blutbestandteile in einem Probenfluid aus Blut des Patienten durch Zugabe eines Reagenzes chemisch nachzuweisen. Deren bloße Existenz - selbst in der erforderlichen Konzentration - lässt allerdings keine verlässliche Aussage darüber zu, inwieweit sie tatsächlich eine funktionale Blutgerinnung bewirken können, um getrennte Gefäße miteinander zu verbinden oder eröffnete Gefäße mechanisch belastbar zu verschließen.

Bei einem verbesserten Verfahren wird zur Messung der Blutgerinnung eine Vorrichtung mit einem Kolben und einem Probengefäß verwendet. Der Kolben ist relativ zu dem Probengefäß derart angeordnet, dass er in das Probengefäß hineinragt. Dem Kolben und/oder dem Probengefäß ist eine Antriebseinheit zugeordnet, die den Kolben und das Probengefäß relativ zueinander bewegt. Zur Messung der Blutgerinnung wird ein Probenfluid aus Blut des Patienten in das Probengefäß eingebracht, so dass der Kolben teilweise in das Probenfluid eingetaucht und von diesem umgeben ist. Dann werden der Kolben und das Probengefäß mittels der Antriebseinheit relativ zu einander zumeist drehend bewegt, wobei die dazu erforderliche und von der Antriebseinheit ausgeübte Kraft fortlaufend gemessen wird. Wegen der infolge der Bewegung auf das Probenfluid wirkenden Scherkräfte setzt ein natürlicher Gerinnungsvorgang ein, der zu einer sich allmählich ausbildenden Vernetzung zwischen dem Kolben und dem Probengefäß führt.

Die zunehmend stabiler werdende Verbindung zwischen dem Kolben und dem Probengefäß erfordert zur Aufrechterhaltung der Relativbewegung zwischen dem Kolben und dem Gefäß eine in gleichem Maße zunehmende Kraft. Trägt man die zu der relativen Drehbewegung erforderliche Kraft gegen die Messzeit auf, ergibt sich bei normaler Blutgerinnung ein typischer Kurvenverlauf (Thrombelastogramm, TEG), während eine gestörte Blutgerinnung zu abweichenden Messkurven führt. Dieses Verfahren ist jedoch wenig empfindlich und kann prinzipiell unterscheidbare funktionale Störungen der Blutgerinnung nicht ausreichend voneinander trennen, wodurch eine zielgerichtete Medikamentengabe erschwert ist.

Ein alternatives, weiter verbessertes Verfahren misst statt der Antriebskraft die Schwingungsamplitude eines Schwingelements (Resonanzthrombelastografie). Eine entsprechende Vorrichtung umfasst statt eines Kolbens ein Schwingelement, dem eine Anregungseinheit zugeordnet ist. Das Schwingelement ist während der Messung teilweise in ein mit einem Probenfluid gefülltes Probengefäß eingetaucht und wird durch die Anregungseinheit mit einer periodischen Kraft derart beaufschlagt, dass es eine erzwungene Schwingung ausführt. Dabei liegt die Anregungsfrequenz knapp oberhalb der Eigenfrequenz des in ungeronnenes Probenfluid eingetauchten Schwingelements. Die durch das Schwingelement auf das Probenfluid ausgeübten Scherkräfte lösen den natürlichen Gerinnungsprozess aus, wodurch sich zwischen dem Schwingelement und dem Probengefäß eine sich allmählich verfestigende Vernetzung ausbildet. Die natürliche Blutgerinnung erhöht daher die Festigkeit des eingetauchten Schwingelements und dementsprechend seine Eigenfrequenz. Wenn die Eigenfrequenz des eingetauchten Schwingelements sich der Anregungsfrequenz von unten nähert, nimmt die Amplitude seiner Schwingung zu, bis sie bei einer Übereinstimmung ihren Maximalwert erreicht (Resonanz). Wenn sich die Eigenfrequenz des eingetauchten Schwingelements dann nach oben von der Anregungsfrequenz entfernt, verringert sich die Amplitude der Schwingung wieder. Trägt man die Schwingungsamplitude als Funktion der Messzeit auf, erhält man eine typische Resonanzkurve.

Das Verhältnis zwischen der Resonanzfrequenz des eingetauchten Schwingelements und dem Frequenzbereich, innerhalb dessen die Schwingung des eingetauchten Schwingelements um weniger als 3 dB gegenüber der Resonanzamplitude gedämpft ist, wird als Güte des eingetauchten Schwingelements bezeichnet. Diese Güte, die bei diesem Verfahren der zeitlichen Ausgeprägtheit der Resonanzkurve entspricht, lässt sich durch die konstruktive Gestaltung des Schwingelements und des Probengefäßes derart beeinflussen, dass mit diesem Verfahren eine äußerst empfindliche und präzise Messung der Blutgerinnung und eine aussagekräftige Darstellung in Form eines Kurvenverlaufs möglich sind.

Für eine verlässliche Messung der Blutgerinnung nach diesem Verfahren ist es allerdings erforderlich, das Volumen des in dem Probengefäß vorhandenen Probenfluids äußerst präzise vorzusehen. Dies erfordert einen durch lange Übung erfahrenen Umgang mit Hochpräzisionspipetten. Ein solcher ist jedoch nicht mehr marktgerecht und bei dem im Operationssaal gegenwärtigen Personal teilweise nicht vorhanden und auch nicht gewünscht. Zudem eignet sich dieses Verfahren nicht für den mobilen notärztlichen Einsatz. Zur Überwindung des letztgenannten Nachteils kann statt eines Probengefäßes eine Kartusche mit einem darin ausgebildeten Probenraum verwendet werden. Beispielsweise offenbart die WO 00/31529 zur ultraschallbasierten Messung der Viskosität eines Probenfluids eine Kartusche mit einem in den Probenraum vorstehenden länglichen Schwingelement.

Ausgehend von diesem Stand der Technik ist es eine Aufgabe der vorliegenden Erfindung, eine Kartusche der eingangs genannten Art sowie eine Vorrichtung zur Koagulationsmessung, in welche die Kartusche einsetzbar ist, zu schaffen, die eine kostengünstige und verlässliche Blutgerinnungsmessung ermöglichen und auch von ungeübtem Personal verwendbar sind.

Zur Lösung der erfindungsgemäßen Aufgabe schafft die vorliegende Erfindung eine Kartusche der eingangs genannten Art, bei welcher der Schwingabschnitt stabförmig mit einer insbesondere rechteckigen oder kreisförmigen Querschnittskontur ausgebildet ist, die quer zu der Längsrichtung des Schwingabschnitts und quer zu der wenigstens einen Schwingrichtung des Schwingabschnitts eine Breite aufweist, die zu einer maximalen Breite des Probenkontaktabschnitts in einem Verhältnis von höchstens 0,2, vorteilhaft höchstens 0,1 steht.

Der Erfindung liegt die Überlegung zugrunde, statt eines Probengefäßes eine Kartusche vorzusehen, innerhalb derer das Probenfluid während der Messung aufgenommen ist. Dazu umfasst die Kartusche ein Gehäuse, in dem ein Probenraum ausgebildet ist, und ein Schwingelement, das mit dem Gehäuse verbunden ist und in dem Probenraum schwingen kann. Das Schwingelement umfasst einen länglichen elastischen Schwingabschnitt, der sich ausgehend von dem Gehäuse in den Probenraum erstreckt, und einen Probenkontaktabschnitt, der an dem freien Ende des Schwingabschnitts ausgebildet und in dem Probenraum angeordnet ist. Ein stabförmiger Schwingabschnitt mit einer rechteckigen oder auch ellipsenförmigen Querschnittskontur eignet sich für eine lineare Schwingung, bei der sich das Schwingelement ausschließlich innerhalb einer Ebene bewegt. Eine kreisförmige Querschnittskontur lässt dagegen eine symmetrische zweidimensionale Schwingung zu, bei der sich das Schwingelement wie bei einer Rührbewegung näherungsweise auf einem Kegelmantel bewegt. Quer zu der Längsrichtung des Schwingabschnitts und quer zu der wenigstens einen Schwingrichtung besitzt der Schwingabschnitt eine Breite, die höchstens ein Fünftel, vorteilhaft höchstens ein Zehntel der entsprechenden Breite des Probenkontaktabschnitts beträgt. Mit anderen Worten ist der Schwingabschnitt relativ zu dem Probenkontaktabschnitt sehr dünn, was mit einer geringen Dämpfung des Schwingelements in dem Probenfluid einhergeht. Durch diese Ausbildung des Schwingelements lässt sich daher eine entsprechend hohe Güte erreichen. Die Menge eines in der Kartusche aufgenommenen Probenfluids ist bestimmt, wenn das Probenfluid den Probenraum vollständig ausfüllt. Auf diese Weise ist das Schwingelement stets von derselben bekannten Menge an Probenfluid umgeben, wodurch reproduzierbare Messbedingungen geschaffen und Fehlerquellen ausgeschlossen sind. In dem Probenraum ist das Probenfluid zudem vor Umwelteinflüssen geschützt. Die Handhabung der Kartusche ist auch ungeübten Personen leicht möglich, da sich der Probenraum mittels einer gewöhnlichen Spritze ohne weiteres vollständig füllen lässt.

Gemäß einer erfindungsgemäßen Ausgestaltung besitzt das von dem ungeronnenen Probenfluid umgebene Schwingelement eine Eigenfrequenz, die in einem Bereich von 20 Hz bis 80 Hz, vorteilhaft zwischen 30 Hz und 50 Hz, vorteilhafter zwischen 35 Hz und 45 Hz liegt und bevorzugt 38 Hz beträgt, und/oder eine Güte, die in einem Bereich von 5 bis 45 liegt und bevorzugt wenigstens 20 beträgt. Eigenfrequenzen in diesem Bereich korrespondieren besonders gut zu den natürlichen Bedingungen der Blutzirkulation in den Blutgefäßen. Güten von 20 oder darüber erlauben eine empfindliche und hochpräzise Messung eines Koagulationsverlaufs.

Vorteilhaft ist der Probenkontaktabschnitt als eine insbesondere rechteckige Platte ausgebildet, deren Plattenlänge sich in der Längsrichtung des Schwingabschnitts und deren Plattenbreite sich quer zu der Längsrichtung des Schwingabschnitts und quer zu der wenigstens einen Schwingrichtung erstreckt, und die an ihren Hauptflächen zwei gegenüberliegend angeordnete Haftflächen definiert, wobei das Verhältnis der Plattenbreite zu der Plattenlänge wenigstens 1, vorteilhaft wenigstens 2 beträgt. Ein plattenförmiger Probenkontaktabschnitt fördert eine flache Ausgestaltung der Kartusche. Ferner ist ein Probenkontaktabschnitt in rechteckiger Gestalt einfach herstellbar und bietet eine große Oberfläche, um Scherkräfte auf das Probenfluid auszuüben und eine Adhäsion von Fibrinfäden zu begünstigen.

Alternativ dazu ist der Probenkontaktabschnitt als ein Zylinder ausgebildet, dessen Zylinderachse sich in der Längsrichtung des Schwingabschnitts erstreckt und der die Haftfläche an einer Außenseite des Zylindermantels definiert, wobei das Verhältnis der Zylinderlänge zu dem Zylinderdurchmesser wenigstens 1, vorteilhaft wenigstens 2 beträgt. Ein zylinderförmiger Probenkontaktabschnitt eignet sich besonders in Kombination mit einem rundstabförmigen Schwingabschnitt, wenn sich das Schwingelement wie bei einer Rührbewegung näherungsweise auf einem Kegelmantel bewegt.

In einer erfindungsgemäßen Variante ist das Schwingelement einteilig mit dem Gehäuse ausgebildet. Dies führt zu einem einfachen Aufbau und einer einfachen Handhabung der Kartusche. Beispielsweise kann das Schwingelement an das Gehäuse angegossen sein, wenn das Schwingelement und das Gehäuse aus Kunststoff hergestellt sind. Dadurch wird ein Übergang zur Mikrochiptechnik eröffnet.

Alternativ kann an dem Gehäuse eine Halterung ausgebildet sein, in welcher ein Befestigungsabschnitt des Schwingelements, der insbesondere gegenüberliegend zu dem Probenkontaktabschnitt an dem Schwingabschnitt des Schwingelements ausgebildet ist, befestigbar oder befestigt.

Bevorzugt weist die Halterung eine Aufnahme, die korrespondierend zu dem Befestigungsabschnitt des Schwingelements ausgebildet ist und in welcher der Befestigungsabschnitt des Schwingelements aufgenommen und positioniert ist, und Klemmmittel auf, die den Befestigungsabschnitt in der Aufnahme fixieren. Eine Halterung mit Aufnahme und Klemmmitteln erlaubt eine einfache Befestigung des Schwingelements an dem Gehäuse der Kartusche. Die Aufnahme und die Klemmmittel stellen sicher, dass das Schwingelement in Bezug auf das Gehäuse stets in einer bestimmten Position festgelegt ist und diese Position während der Messung beibehält.

Gemäß einer vorteilhaften Weiterbildung der Kartusche umfasst das Gehäuse einen Gehäusekörper, in dem der Probenraum und die Halterung ausgebildet sind, und einen den Probenraum und die Aufnahme verschließenden Deckel, an dem wenigstens ein Klemmelement, insbesondere ein in Richtung der Aufnahme vorstehender Klemmvorsprung derart vorgesehen ist, dass der Befestigungsabschnitt des Schwingelements zwischen einem Boden der Aufnahme und dem Klemmvorsprung eingeklemmt ist, wenn der Deckel den Gehäusekörper verschließt, wobei eine maximale Länge der Kartusche in dem Bereich von 20 mm bis 80 mm, vorteilhaft zwischen 40 mm und 60 mm liegt und bevorzugt 50 beträgt. Ein derartiges Gehäuse erlaubt das Positionieren des Befestigungsabschnitts des Schwingelements in der Aufnahme, bevor es beim Verschließen des Gehäusekörpers mit dem Deckel automatisch in der Halterung festgeklemmt wird. Dies ist eine besonders einfache Ausgestaltung einer Klemmverbindung, eine kostengünstige und leicht handhabbare Befestigung des Schwingelements an dem Gehäuse erlaubt. Die angegebene Längenausdehnung der Kartusche erleichtert die Handhabung der Kartusche unter Einsatzbedingungen.

Bevorzugt ist der Befestigungsabschnitt des Schwingelements plattenförmig ausgebildet und besitzt insbesondere eine rechteckige Gestalt. Eine plattenförmige Ausbildung des Befestigungsabschnitts erlaubt eine flache Ausbildung der Kartusche. Ein Befestigungsabschnitt mit rechteckiger Gestalt lässt sich zudem besonders einfach in einer Aufnahme einer Halterung positionieren. Derartige Befestigungsabschnitte sind beispielsweise aus einem Stahlblech einfach und kostengünstig herstellbar.

Gemäß einer Weiterbildung ist an dem Probenkontaktabschnitt eine elektrisch leitende Kondensatorfläche ausgebildet, die mit einem an einer Außenseite des Gehäuses vorgesehenen Kontaktbereich elektrisch leitend verbunden ist. Eine derartige elektrisch leidende Kondensatorfläche kann verwendet werden, um zusammen mit außerhalb der Kartusche angeordneten Kondensatorplatten die Position des Probenkontaktabschnitts durch eine Kapazitätsmessung zu bestimmen. Die zur Verbindung mit der Kontaktfläche erforderliche elektrische Leitfähigkeit des Schwingelements kann beispielsweise durch Bedampfen mit einem elektrisch leitenden Material hergestellt sein.

Bevorzugt weist der Probenkontaktabschnitt magnetisierbares oder magnetisches Material auf. Ein solcher Probenkontaktabschnitt lässt sich berührungslos durch ein magnetisches Wechselfeld zu Schwingungen anregen und ermöglicht einen einfachen und geschlossenen Aufbau der Kartusche.

Gemäß einer Variante der Kartusche weist der Probenkontaktabschnitt Stahl auf oder besteht daraus. Stahl ist ein magnetisierbares Material, dass in Form von Stahlblech einfach und kostengünstig zur Herstellung plattenförmiger Probenkontaktabschnitte verwendet werden kann.

An einer Haftfläche des Probenkontaktabschnittes kann eine Kunststoffbeschichtung vorgesehen sein, die bezogen auf das Probenfluid bessere Adhäsionseigenschaften als das Material der Oberfläche des Probenkontaktabschnitts aufweist. Eine derartige Beschichtung begünstigt die Anhaftung des gerinnenden Probenfluids.

Alternativ kann der Probenkontaktabschnitt aus Kunststoff hergestellt sein und einen Permanentmagneten tragen. In dieser Ausgestaltung wird also der Probenkontaktabschnitt mit magnetischem Material in Form eines Permanentmagneten versehen. Es wäre aber auch ausreichend, magnetisierbares Material vorzusehen.

Gemäß einer Weiterentwicklung der vorliegenden Erfindung ist das Schwingelement in seiner Längsrichtung in mehrere Schwingelementabschnitte unterteilt, wobei die Schwingelementabschnitte insbesondere durch eine Steckverbindung miteinander verbindbar oder verbunden sind. Dieser Aufbau eines Schwingelements kann die Herstellung des Schwingelements vereinfachen.

Vorteilhaft umfassen die Schwingelementabschnitte den Schwingabschnitt, den Probenkontaktabschnitt und/oder insbesondere den Befestigungsabschnitt und sind aus unterschiedlichen Materialien hergestellt. Auf diese Weise lassen sich Schwingelemente flexibel herstellen und zusammensetzen.

Bevorzugt weist das Gehäuse einen Einfüllkanal auf, der sich ausgehend von einer in einer Wand des Gehäuses vorgesehenen Einfüllöffnung in den Probenraum erstreckt und dem ein Einfüllkanalverschlusselement, insbesondere ein Durchstechventil zugeordnet ist. Ein in dem Gehäuse vorgesehener Einfüllkanal verbindet die Außenseite der Kartusche mit dem Probenraum derart, dass dem Probenraum durch diesen einfach mittels einer Spritze Probenfluid zugeführt werden kann.

Bevorzugt weist das Gehäuse einen Überlaufkanal auf, der sich ausgehend von einer in einer Wand des Gehäuses vorgesehenen Überlauföffnung in den Probenraum erstreckt, wobei dem Überlaufkanal ein Überlaufkanalverschlusselement, insbesondere eine hydrophobe und/oder hydrophile Fritte oder eine gasdurchlässige Membran zugeordnet ist. Durch einen Überlaufkanal kann die in der Kartusche enthaltene Luft entweichen, wenn durch den Einfüllkanal Blut in den Probenraum eingefüllt wird. Dagegen wird das Probenfluid durch das Überlaufkanalverschlusselement daran gehindert, aus der Kartusche auszutreten.

Vorteilhaft münden der Einfüllkanal und der Überlaufkanal in gegenüberliegende Bereiche des Probenraums. Infolgedessen fließt Blut in den Überlaufkanal, wenn der Probenraum vollständig mit Blut gefüllt ist. Aus dem Überlaufkanal austretendes Blut kann daher eine vollständige Füllung des Probenraums mit Blut anzeigen.

Der Überlaufkanal kann vorteilhaft einen insbesondere mäanderförmigen Kontrollabschnitt aufweisen, der durch einen transparenten Abschnitt des Gehäuses von außen optisch zugänglich ist. Wenn das in den Probenraum eingefüllte Probenfluid in den mäanderförmigen Abschnitt des Überlaufkanals gelangt, kann die zu diesem Zeitpunkt bereits ausreichende Befüllung des Probenraums mit Blut optisch einfach erkannt werden und das Einfüllen von Blut beendet werden, ohne dass Blut aus der Überlauföffnung austritt. Eine Möglichkeit besteht in der visuellen Überwachung des Kontrollabschnitts durch die das Probenfluid einfüllende Person. Alternativ kann aber auch ein optischer Füllstandsensor verwendet werden, der den Kontrollabschnitt einer in eine Vorrichtung zur Koagulationsmessung eingesetzten Kartusche beim Einfüllen des Probenfluids überwacht und der einfüllenden Person ein Signal gibt, sobald Probenfluid in den Kontrollabschnitt einströmt.

Gemäß einer Weiterbildung ist an einer Außenseite des Gehäuses eine Markierung vorgesehen ist, die insbesondere als Markierungsvorsprung ausgebildet ist, der von dem Gehäuse auswärts vorsteht. Eine solche Markierung kann von einer entsprechenden Vorrichtung zur Koagulationsmessung beispielsweise verwendet werden, um die Anwesenheit einer Kartusche mechanisch oder auf andere Weise zu detektieren und automatisch einen Messvorgang zu beginnen, wenn eine Kartusche anwesend ist. Alternativ oder zusätzlich kann die Markierung zusammen mit weiteren Kartuscheninformationen zur Qualitätssicherung beitragen, indem beispielsweise Kartuschen, deren Mindesthaltbarkeitsdatum überschritten ist, zum Zeitpunkt der Messung von der Vorrichtung zur Koagulationsmessung nicht mehr akzeptiert werden.

Bei einer erfindungsgemäßen Kartusche kann der Markierungsvorsprung an dem Befestigungsabschnitt des Schwingelements, insbesondere gegenüberliegend zu dem Schwingabschnitt ausgebildet sein und einen in dem Gehäuse vorgesehenen Durchlass durchsetzen. Ein das Gehäuse durchsetzender Markierungsvorsprung kann elektrisch leitend mit dem Probenkontaktabschnitt des Schwingelements verbunden sein und als elektrischer Kontaktbereich der Kartusche für die kapazitative Messung der Position des Probenkontaktbereichs dienen.

Bevorzugt bildet der Markierungsvorsprung des Schwingelements gleichzeitig den außenseitigen Kontaktbereich einer an dem Probenkontaktbereich vorgesehenen Kondensatorfläche. Auf diese Weise kann die Markierung der Kartusche gleichzeitig zwei Funktionen übernehmen.

Vorteilhaft ist innerhalb der Kartusche ein Reagenz vorgesehen, das insbesondere als eine an dem Probenkontaktabschnitt des Schwingelements und/oder an einer Randfläche des Probenraums vorgesehene und in dem Probenfluid lösliche Beschichtung und/oder als eine mit dem Probenfluid mischbare Flüssigkeit, die in einer in dem Gehäuse ausgebildeten und mit dem Probenraum verbundenen Reagenzkammer enthalten ist, vorgesehen ist. Reagenzien können das in dem Probenraum aufgenommenen Probefluid derart beeinflussen, dass eine Messung einzelner Gerinnungsfunktionen und/oder -faktoren möglich ist. Wenn das Reagenz in flüssiger Form, bevorzugt als Lyophilisat in einer Reagenzkammer vorgesehen ist, kann die Reagenzkammer beispielsweise als Teil des Einfüllkanals derart ausgebildet sein, dass das Probenfluid die Reagenzkammer bei dem Einfüllen durchströmt und sich dabei mit dem Reagenz vermischt. Selbstverständlich ist es alternativ möglich, ein flüssiges Reagenz beispielsweise mittels einer Spritze vor oder nach dem Probenfluid durch die Einfüllöffnung in den Probenraum einzufüllen.

Gemäß einer Weiterbildung ist an einer Außenfläche des Gehäuses wenigstens eine Identifikationseinrichtung vorgesehen, die insbesondere als Barcode oder als RFID-Chip ausgebildet ist. Eine Identifikationseinrichtung ermöglicht eine einfache Identifikation der damit versehenen Kartusche hinsichtlich einer Herkunft des Probenfluids wie beispielsweise der Identität eines Patienten und/oder in der Kartusche vorgesehener Reagenzien. Eine Ausbildung der Identifikationseinrichtung als Barcode erlaubt eine einfache und kostengünstige automatische Identifikation einer zu vermessenden Kartusche durch eine entsprechende Vorrichtung zur Koagulationsmessung, in welche die Kartusche eingesetzt ist.

Weiterhin schafft die vorliegende Erfindung eine Vorrichtung zur Koagulationsmessung mittels einer erfindungsgemäßen Kartusche, mit wenigstens einer Kartuschenaufnahme, in welche die Kartusche einsetzbar ist, einer Anregungseinheit, die ausgebildet und angeordnet ist, um das Schwingelement einer in die Kartuschenaufnahme eingesetzten Kartusche zum Schwingen mit einer Schwingamplitude anzuregen, einer Sensoreinheit, die ausgebildet und angeordnet ist, um die Schwingamplitude des Schwingelements zu messen, wobei die Anregungseinheit und die Sensoreinheit jeweils der wenigstens einen Kartuschenaufnahme zugeordnet und zur automatischen Durchführung einer Koagulationsmessung mit einer Steuereinheit verbunden sind, wobei die Anregungseinheit ausgelegt ist, um Anregungsfrequenzen oberhalb der Eigenfrequenz des Schwingelements derart zu erzeugen, dass die Differenz zwischen der Anregungsfrequenz und der Eigenfrequenz in einem Bereich von 1 Hz bis 10 Hz, vorteilhaft zwischen 2 Hz und 6 Hz liegt und bevorzugt 4 Hz beträgt, und Schwingamplituden des Schwingelements in einem Bereich von 0,5 mm bis 2,5 mm, vorteilhaft zwischen 1 mm und 2 mm und bevorzugt von 1,5 mm hervorzurufen.

Der Erfindung liegt die Überlegung zugrunde, eine Vorrichtung zur Koagulationsmessung nach dem lab-on-the-chip-Konzept zu schaffen, die ausgebildet ist, um die Gerinnung eines in einer Kartusche enthaltenen Probenfluids zu messen. Dazu umfasst die Vorrichtung wenigstens eine Kartuschenaufnahme, in welche die Kartusche einsetzbar ist und welcher eine Anregungseinheit sowie eine Sensoreinheit zugeordnet sind. Die Anregungseinheit regt das in der Kartusche angeordnete und von dem Probenfluid umgebene Schwingelement zu einer erzwungenen Schwingung an, während die Sensoreinheit die Amplitude der Schwingung misst. Bei fortschreitender Gerinnung des Probenfluids verändert sich das Schwingungsverhalten des Schwingelements, was bei konstanter Anregungsfrequenz beispielsweise als Amplitudenveränderung der Schwingung messbar ist. Dabei ist die konstante Anregungsfrequenz derart gewählt, dass sie wenig oberhalb der Eigenfrequenz des Schwingelements liegt. Mit fortschreitender Koagulation des Probenfluids erhöht sich die Eigenfrequenz des Schwingelements, wobei die Schwingamplitude durch ein Maximum läuft, wenn die Eigenfrequenz mit der Anregungsfrequenz übereinstimmt. Die Anregungsstärke ist derart gewählt, dass sich eine Schwingamplitude von wenigen Millimetern einstellt. Anregungsfrequenzen und Schwingamplituden dieser Größenordnungen können die natürlichen Bedingungen der Blutzirkulation in den Blutgefäßen besonders gut simulieren.

Gemäß einer Weiterbildung der vorliegenden Erfindung umfasst die Anregungseinheit wenigstens zwei elektrisch erregbare Magnetspulen, die benachbart zu der wenigstens einen Kartuschenaufnahme derart ausgebildet und angeordnet sind, dass sie den Probenkontaktabschnitt des Schwingelements der eingesetzten Kartusche wahlweise mit einer Magnetkraft beaufschlagen. Die wenigstens zwei elektrisch erregbaren Magnetspulen erzeugen ein magnetisches Wechselfeld, in welchem das magnetische oder magnetisierbare Material des Probenkontaktabschnittes mit einer hinsichtlich Richtung und Stärke variablen Kraft beaufschlagt werden kann.

Die Anregungseinheit umfasst gemäß einer Variante genau zwei Magnetspulen, die auf gegenüberliegenden Seiten der Kartuschenaufnahme angeordnet sind. Diese Anordnung genau zweier Magnetspulen eignet sich für eine lineare Anregung des Schwingelements.

Alternativ kann die Anregungseinheit genau vier Magnetspulen umfassen, die in gleichen Winkelabständen um die Kartuschenaufnahme angeordnet sind. Auf diese Weise lässt sich das Schwingelement in der Kartusche zu einer zweidimensionalen kreisförmigen Schwingbewegung ähnlich einer Rührbewegung in dem Probenfluid anregen. Eine solche Bewegung des Schwingelements weist keine Umkehrpunkte auf und entspricht in hohem Maße den natürlichen Verhältnissen in den Blutgefäßen.

Gemäß einer Weiterbildung der erfindungsgemäßen Vorrichtung umfasst die Sensoreinheit wenigstens eine Kondensatorplatte, die sich parallel zu einem Probenkontaktabschnitt eines Schwingelements einer in die Kartuschenaufnahme eingesetzten Kartusche erstreckt. Durch die Schwingbewegung einer des Probenkontaktabschnitts verändert sich der Abstand zwischen der Kondensatorplatte und einer an dem Probenkontaktabschnitt ausgebildeten elektrischen Leitungsplatte. Dies bewirkt eine messbare Kapazitätsänderung des aus der Kondensatorplatte und der elektrischen Leitungsplatte an dem Probenkontaktabschnitt gebildeten Kondensators, die mit der Schwingbewegung des Probenkontaktabschnitts unmittelbar korreliert. Gemäß einer Variante sind zwei Kondensatorplatten auf gegenüberliegenden Seiten der Kartuschenaufnahme angeordnet, um zwei Kondensatoren zu bilden, deren Kapazitäten sich infolge der Schwingbewegung des Probenkontaktabschnitts jeweils gegenläufig verändern, wodurch sich die Messgenauigkeit erhöhen kann, insbesondere bei unsymmetrischer Koagulation bezogen auf die Schwingbewegung des Schwingelements. Alternativ kann die Sensoreinheit auch einen Reflexsensor oder einen Hallsensor umfassen, um die Schwingbewegung des Schwingelements optisch oder magnetisch zu erfassen.

Vorteilhaft ist der wenigstens einen Kartuschenaufnahme eine mit der Steuereinheit verbundene Beheizungseinheit zugeordnet, die derart ausgebildet und angeordnet ist, dass das in einer eingesetzten Kartusche enthaltene Probenfluids eine bestimmte Temperatur erreicht und/oder beibehält. Gerinnungsprozesse sind sehr temperaturabhängig. Beispielsweise vollzieht sich die Blutgerinnung typischerweise bei Körpertemperatur. Entsprechend kann eine Beheizungseinrichtung eine Temperatur des Blutes von ca. 37 °C gewährleisten.

Ferner kann der wenigstens einen Kartuschenaufnahme eine mit der Steuereinheit verbundene Füllkontrolleinheit zugeordnet sein, die derart ausgebildet und angeordnet ist, dass sie die Füllung der Kartusche insbesondere optisch erkennt. Als Füllkontrolleinheit können beispielsweise eine Leuchtdiode und ein lichtempfindlicher Widerstand auf gegenüberliegenden Seiten der Kartuschenaufnahme derart angeordnet sein, dass das Licht der Leuchtdiode einen transparenten Bereich eines Gehäuses einer eingesetzten Kartusche durchscheint und die Lichtdurchlässigkeit des Probenraums der Kartusche oder eines Kontrollabschnitts ihres Überlaufkanals messbar macht.

Gemäß einer weiteren Ausgestaltung ist der wenigstens einen Kartuschenaufnahme eine mit der Steuereinheit verbundene Erkennungseinheit zugeordnet, die derart ausgebildet und angeordnet ist, dass sie eine Erkenneinrichtung einer eingesetzten Kartusche erkennt. Auf diese Weise lässt sich beispielsweise eine Koagulationsmessung automatisch starten, wenn eine mit einer Erkenneinrichtung versehene Kartusche in die Kartuschenaufnahme der Vorrichtung eingesetzt ist.

Gemäß einer Weiterbildung ist der wenigstens eine Kartuschenaufnahme wenigstens eine mit der Steuereinheit verbundene Ableseeinheit zugeordnet, die ausgebildet und angeordnet ist, um eine an der eingesetzten Kartusche befestigte Identifikationseinheit abzulesen. Die Ableseeinheit erlaubt die automatische Zuordnung des in der eingesetzten Kartusche enthaltenen Probenfluids zu einem bestimmten Patienten.

Vorteilhaft ist eine Mehrzahl von Kartuschenaufnahmen, insbesondere zwei Kartuschenaufnahmen vorgesehen, wobei die Vorrichtung um weitere insbesondere zwei Kartuschenaufnahmen erweiterbar sein kann. Eine Vorrichtung mit mehreren Kartuschenaufnahmen, die insbesondere um weitere Kartuschenaufnahmen erweiterbar ist, gestattet eine gleichzeitige Messung mehrerer Probefluide. Dies ist beispielsweise erforderlich, um zu Kontrollzwecken neben dem zu vermessendem Probenfluid ein Kontrollfluid vermessen werden muss.

Weitere Merkmale und Vorteile der vorliegenden Erfindung werden anhand der nachfolgenden Beschreibung verschiedener Ausgestaltungen der erfindungsgemäßen Kartusche unter Bezugnahme auf die beiliegende Zeichnung deutlich. Darin ist:
- Figur 1: eine perspektivische Explosionsansicht einer Kartusche gemäß einer ersten Ausführungsform der vorliegenden Erfindung;
- Figur 2: eine perspektivische Explosionsansicht einer Kartusche gemäß einer zweiten Ausführungsform der vorliegenden Erfindung;
- Figur 3: eine perspektivische Explosionsansicht einer Kartusche gemäß einer dritten Ausführungsform der vorliegenden Erfindung;
- Figur 4: eine perspektivische Explosionsansicht einer Kartusche gemäß einer vierten Ausführungsform der vorliegenden Erfindung;
- Figur 5: eine perspektivische Ansicht einer Kartusche gemäß einer fünften Ausführungsform der vorliegenden Erfindung ohne Deckel;
- Figur 6: eine perspektivische Ansicht einer Kartusche gemäß einer sechsten Ausführungsform der vorliegenden Erfindung ohne Deckel;
- Figur 7: eine perspektivische Ansicht eines Schwingelements einer Kartusche gemäß einer siebten Ausführungsform der vorliegenden Erfindung;
- Figur 8: eine perspektivische Explosionsansicht des in Figur 7 dargestellten Schwingelements;
- Figur 9: eine perspektivische Außenansicht einer Vorrichtung zur Koagulationsmessung gemäß einer ersten Ausführungsform der vorliegenden Erfindung;
- Figur 10: eine perspektivische schematische Innenansicht der in Figur 9 dargestellten Vorrichtung zur Koagulationsmessung;
- Figur 11: eine perspektivische Außenansicht einer Vorrichtung zur Koagulationsmessung gemäß einer zweiten Ausführungsform der vorliegenden Erfindung;
- Figur 12: eine perspektivische Explosionsansicht der in Figur 1 dargestellten Kartusche zusammen mit einer Anregungseinheit und einer Sensoreinheit der in den Figuren 9 und 11 dargestellten Vorrichtungen zur Koagulationsmessung;
- Figur 13: eine perspektivische Ansicht der in Figur 3 dargestellten Kartusche ohne Deckel zusammen mit einer Anregungseinheit und einer Sensoreinheit aus jeweils zwei Magnetspulen bzw. Kondensatorplatten;
- Figur 14: eine perspektivische Ansicht der in Figur 6 dargestellten Kartusche ohne Deckel, jedoch mit dem in den Figuren 7 und 8 dargestellten Schwingelement zusammen mit einer Anregungseinheit aus vier Magnetspulen;
- Figur 15: eine perspektivische Ansicht der mit einer Füllkontrolleinheit der in den Figuren 9 und 11 dargestellten Vorrichtungen zur Koagulationsmessung;
- Figur 16: ein Blockschaltbild der in den Figuren 9 und 11 dargestellten Vorrichtungen zur Koagulationsmessung;
- Figur 17: eine schematische Ansicht einer mit der in den Figuren 9 und 11 dargestellten Vorrichtung nach einem ersten Verfahren gemessenen Messkurve;
- Figur 18: eine erste Messkurve, die mit der in den Figuren 9 und 11 dargestellten Vorrichtung zur Koagulationsmessung gemessen wurde;
- Figur 19: eine zweite Messkurve, die mit der in den Figuren 9 und 11 dargestellten Vorrichtung zur Koagulationsmessung gemessen wurde; und
- Figur 20: eine schematische Ansicht einer mit der in den Figuren 9 und 11 dargestellten Vorrichtung nach einem zweiten Verfahren gemessenen Messkurve.

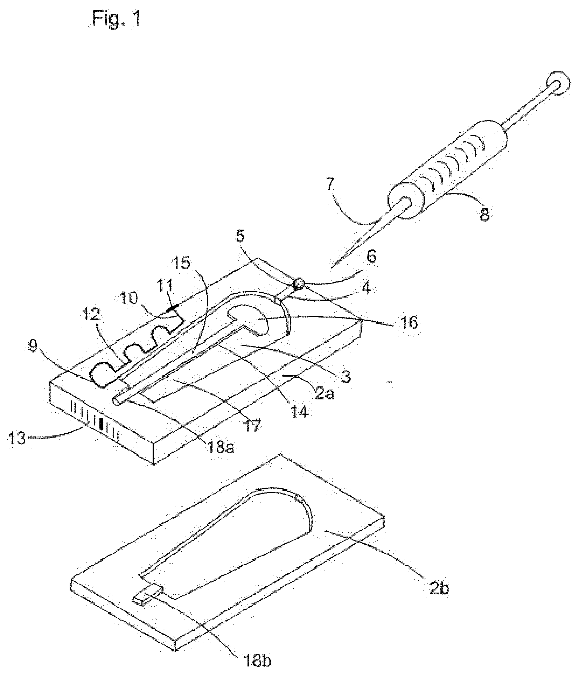

Die Figuren 1 bis 8 zeigen Kartuschen gemäß sieben verschiedener Ausführungsformen der vorliegenden Erfindung zum Einsetzen in eine entsprechende Vorrichtung zur Koagulationsmessung.

Die Figur 1 zeigt eine erste Ausführungsform einer erfindungsgemäßen Kartusche 1. Die Kartusche 1 umfasst ein längliches Gehäuse 2 mit einem Gehäusekörper 2a und einem Deckel 2b, die jeweils eine Länge von ungefähr 50 mm besitzen. Die Länge des Gehäuses kann zwischen 20 mm und 80 mm liegen. In dem Gehäuse 2 ist ein Probenraum 3 zum Aufnehmen eines Probenfluids ausgebildet.

Der Gehäusekörper 2a weist einen Einfüllkanal 4 auf, der sich ausgehend von einer in einer Wand des Gehäusekörpers 2a vorgesehenen Einfüllöffnung 5 in den Probenraum 3 erstreckt. Dem Einfüllkanal 4 ist ein Durchstechventil 6 als Einfüllkanalverschlusselement zugeordnet. Das Durchstechventil 6 ist derart ausgebildet, dass es eine Nadel 7 einer medizinischen Spritze 8 dicht umschließt, die in den Einfüllkanal 4 eingeführt ist.

Weiterhin weist der Gehäusekörper 2a einen Überlaufkanal 9 auf, der sich ausgehend von einer in einer Wand des Gehäusekörpers 2a vorgesehenen Überlauföffnung 10 in den Probenraum 3 erstreckt. Dem Überlaufkanal 9 ist eine hydrophobe und/oder hydrophile Fritte 11 als Überlaufkanalverschlusselement zugeordnet, die derart ausgebildet ist, dass durch sie Luft, nicht aber Probenfluid aus dem Probenraum 3 entweichen kann. Alternativ zu der hydrophoben und/oder hydrophilen Fritte 11 kann dem Überlaufkanal 9 auch eine gasdurchlässige Membran zugeordnet sein. Der Einfüllkanal 4 und der Überlaufkanal 10 münden in gegenüberliegende Bereiche des Probenraums 3, so dass Probenfluid erst dann in den Überlaufkanal 9 gelangt, wenn der Probenraum 3 vollständig mit dem Probenfluid gefüllt ist. Der Überlaufkanal 9 weist einen mäanderförmigen Kontrollabschnitt 12 auf. Dadurch wird eine große Länge des Überlaufkanals 9 erreicht, so dass Probenfluid nicht sofort zu der Überlauföffnung 10 gelangt, wenn der Probenraum 3 vollständig gefüllt ist und weiter Probenfluid zugeführt wird. Im Bereich des mäanderförmigen Kontrollabschnitts 12 des Überlaufkanals 9 weisen der Deckel 2b und der Gehäusekörper 2a transparente Abschnitte derart auf, dass der Kontrollabschnitt 12 von außen optisch zugänglich ist.

An einer Außenfläche des Gehäusekörpers 2a, die gegenüberliegend zu der Einfüllöffnung 5 und der Überlauföffnung 10 angeordnet ist, ist ein Barcode 13 als Identifikationseinrichtung vorgesehen. Alternativ oder zusätzlich kann die Identifikationseinrichtung auch als RFID-Chip ausgebildet sein.

Die Kartusche 1 umfasst ferner ein Schwingelement 14, das einen mit dem Gehäuse 2 verbundenen und von diesem in den Probenraum 3 vorstehenden länglichen, in einer Schwingrichtung elastisch biegbaren Schwingabschnitt 15 aufweist. Der Schwingabschnitt 15 des Schwingelements 14 besitzt eine stabförmige Gestalt und weist quer zu seiner Längsrichtung eine rechteckige Querschnittskontur auf. Die Querschnittskontur weist quer zu der Längsrichtung und quer zu der Schwingrichtung eine Breite auf, die zu der maximalen Breite des Probenkontaktabschnitts in einem Verhältnis von ungefähr 0,2 steht, was mit einer geringen Dämpfung durch das Probenfluid einhergeht und zu einer entsprechend hohen Güte des Schwingelements führt.

An dem freien Ende des Schwingabschnitts 15 ist ein plattenförmiger Probenkontaktabschnitt 16 ausgebildet, der eine ovale Gestalt besitzt und an seinen gegenüberliegenden Hauptflächen zwei Haftflächen definiert. Seine quer zu der Schwingrichtung und quer zu der Längsrichtung gemessene Breite ist ungefähr doppelt so groß wie seine in der Längsrichtung gemessene Länge. Gegenüberliegend zu dem Probenkontaktabschnitt 16 ist an dem Schwingabschnitt 15 ein Befestigungsabschnitt 17 ausgebildet. Der Befestigungsabschnitt 17 ist plattenförmig ausgebildet und besitzt eine rechteckige Gestalt. Der Schwingabschnitt 15, der Probenkontaktabschnitt 16 sowie der Befestigungsabschnitt 17 des Schwingelements 14 sind einteilig aus Stahl hergestellt, so dass insbesondere der Probenkontaktabschnitt 16 aus einem magnetischen Material besteht und eine elektrisch leitende Kondensatorfläche bildet, die mit einem an der Außenseite des Gehäusekörpers 2a vorgesehenen Kontaktbereich elektrisch leitend verbunden ist. Die Eigenfrequenz des von dem ungeronnenen Probenfluid umgebenen Schwingelements 14 liegt bei ungefähr 38 Hz. Die Güte beträgt ca. 20.

Zur Befestigung des Schwingelements 14 ist in dem Gehäuse 2 eine Halterung 18 ausgebildet, in welcher der Befestigungsabschnitt 17 aufgenommen und positioniert ist. Die Halterung 18 weist eine zu dem Befestigungsabschnitt 17 korrespondierende Aufnahme 18a auf, die in dem Gehäusekörper 2a ausgebildet ist und in welcher der Befestigungsabschnitt 17 des Schwingelements 14 aufgenommen und positioniert ist. Ferner weist die Halterung 18 einen Klemmvorsprung 18b auf, der an dem Deckel 2b vorgesehen ist und in Richtung der Aufnahme 18a vorsteht, so dass der Befestigungsabschnitt 17 zwischen einem Boden der Aufnahme 18a und dem Klemmelement 18b eingeklemmt ist, wenn der Deckel 2b den Probenraum 3 und die Aufnahme 18a verschließt.

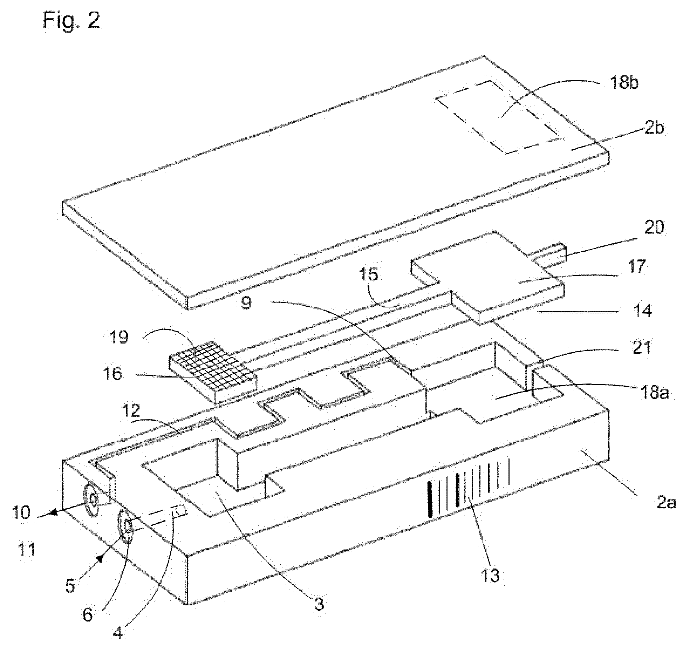

Die Figur 2 zeigt eine zweite Ausführungsform einer erfindungsgemäßen Kartusche 1, die im Wesentlichen denselben Aufbau wie die in Figur 1 dargestellte Kartusche aufweist, jedoch zusätzlich eine Haftbeschichtung 19 sowie einen Markierungsvorsprung 20 sowie einen Durchlass 21 aufweist.

Die Kartusche 1 umfasst ein längliches Gehäuse 2 mit einem Gehäusekörper 2a und einem Deckel 2b, die jeweils eine Länge von ungefähr 50 mm besitzen. Die Länge des Gehäuses kann zwischen 20 mm und 80 mm liegen. In dem Gehäuse 2 ist ein Probenraum 3 zum Aufnehmen eines Probenfluids ausgebildet.

Der Gehäusekörper 2a weist einen Einfüllkanal 4 auf, der sich ausgehend von einer in einer Wand des Gehäusekörpers 2a vorgesehenen Einfüllöffnung 5 in den Probenraum 3 erstreckt. Dem Einfüllkanal 4 ist ein Durchstechventil 6 als Einfüllkanalverschlusselement zugeordnet. Das Durchstechventil 6 ist derart ausgebildet, dass es eine Nadel 7 einer medizinischen Spritze 8 dicht umschließt, die in den Einfüllkanal 4 eingeführt ist.

Weiterhin weist der Gehäusekörper 2a einen Überlaufkanal 9 auf, der sich ausgehend von einer in einer Wand des Gehäusekörpers 2a vorgesehenen Überlauföffnung 10 in den Probenraum 3 erstreckt. Dem Überlaufkanal 9 ist eine hydrophobe und/oder hydrophile Fritte 11 als Überlaufkanalverschlusselement zugeordnet, die derart ausgebildet ist, dass durch sie Luft, nicht aber Probenfluid aus dem Probenraum 3 entweichen kann. Alternativ zu der hydrophoben und/oder hydrophilen Fritte 11 kann dem Überlaufkanal 9 auch eine gasdurchlässige Membran zugeordnet sein. Der Einfüllkanal 4 und der Überlaufkanal 10 münden in gegenüberliegende Bereiche des Probenraums 3, so dass Probenfluid erst dann in den Überlaufkanal 9 gelangt, wenn der Probenraum 3 vollständig mit dem Probenfluid gefüllt ist. Der Überlaufkanal 9 weist einen mäanderförmigen Kontrollabschnitt 12 auf. Dadurch wird eine große Länge des Überlaufkanals 9 erreicht, so dass Probenfluid nicht sofort zu der Überlauföffnung 10 gelangt, wenn der Probenraum 3 vollständig gefüllt ist und weiter Probenfluid zugeführt wird. Im Bereich des mäanderförmigen Kontrollabschnitts 12 des Überlaufkanals 9 weisen der Deckel 2b und der Gehäusekörper 2a transparente Abschnitte derart auf, dass der Kontrollabschnitt 12 von außen optisch zugänglich ist.

An einer Außenfläche des Gehäusekörpers 2a, die gegenüberliegend zu der Einfüllöffnung 5 und der Überlauföffnung 10 angeordnet ist, ist ein Barcode 13 als Identifikationseinrichtung vorgesehen. Alternativ oder zusätzlich kann die Identifikationseinrichtung auch als RFID-Chip ausgebildet sein.

Die Kartusche 1 umfasst ferner ein Schwingelement 14, das einen mit dem Gehäuse 2 verbundenen und von diesem in den Probenraum 3 vorstehenden länglichen, in einer Schwingrichtung elastisch biegbaren Schwingabschnitt 15 aufweist. Der Schwingabschnitt 15 des Schwingelements 14 besitzt eine stabförmige Gestalt und weist quer zu seiner Längsrichtung eine rechteckige Querschnittskontur auf. Die Querschnittskontur weist quer zu der Längsrichtung und quer zu der Schwingrichtung eine Breite auf, die zu der maximalen Breite des Probenkontaktabschnitts in einem Verhältnis von ungefähr 0,2 steht, was mit einer geringen Dämpfung durch das Probenfluid einhergeht und zu einer entsprechend hohen Güte des Schwingelements führt.

An dem freien Ende des Schwingabschnitts 15 ist ein plattenförmiger Probenkontaktabschnitt 16 ausgebildet, der eine rechteckige Gestalt besitzt und an seinen gegenüberliegenden Hauptflächen zwei Haftflächen definiert. Seine quer zu der Schwingrichtung und quer zu der Längsrichtung gemessene Breite ist ungefähr doppelt so groß wie seine in der Längsrichtung gemessene Länge. Gegenüberliegend zu dem Probenkontaktabschnitt 16 ist an dem Schwingabschnitt 15 ein Befestigungsabschnitt 17 ausgebildet. Der Befestigungsabschnitt 17 ist plattenförmig ausgebildet und besitzt eine rechteckige Gestalt. Der Schwingabschnitt 15, der Probenkontaktabschnitt 16 sowie der Befestigungsabschnitt 17 des Schwingelements 14 sind einteilig aus Stahl hergestellt, so dass insbesondere der Probenkontaktabschnitt 16 aus einem magnetischen Material besteht und eine elektrisch leitende Kondensatorfläche bildet, die mit einem an der Außenseite des Gehäusekörpers 2a vorgesehenen Kontaktbereich elektrisch leitend verbunden ist. An den Haftflächen des Probenkontaktabschnitts 16 ist eine Haftbeschichtung 19 aus Kunststoff ausgebildet, die bezogen auf das Probenfluid bessere Adhäsionseigenschaften als Stahl aufweist. Die Eigenfrequenz des von dem ungeronnenen Probenfluid umgebenen Schwingelements 14 liegt bei ungefähr 38 Hz. Die Güte beträgt ca. 20.

Zur Befestigung des Schwingelements 14 ist in dem Gehäuse 2 eine Halterung 18 ausgebildet, in welcher der Befestigungsabschnitt 17 aufgenommen und positioniert ist. Die Halterung 18 weist eine zu dem Befestigungsabschnitt 17 korrespondierende Aufnahme 18a auf, die in dem Gehäusekörper 2a ausgebildet ist und in welcher der Befestigungsabschnitt 17 des Schwingelements 14 aufgenommen und positioniert ist. Ferner weist die Halterung 18 einen Klemmvorsprung 18b auf, der an dem Deckel 2b vorgesehen ist und in Richtung der Aufnahme 18a vorsteht, so dass der Befestigungsabschnitt 17 zwischen einem Boden der Aufnahme 18a und dem Klemmelement 18b eingeklemmt ist, wenn der Deckel 2b den Probenraum 3 und die Aufnahme 18a verschließt. An dem Befestigungsabschnitt 17 ist gegenüberliegend zu dem Schwingabschnitt 15 ein Markierungsvorsprung 20 ausgebildet, der einen in dem Gehäusekörper 2a vorgesehenen Durchlass 21 durchsetzt. Der Markierungsvorsprung 20 bildet gleichzeitig den Kontaktbereich der Kondensatorfläche des Probenkontaktabschnitts 16 an der Außenseite des Gehäuses 2.

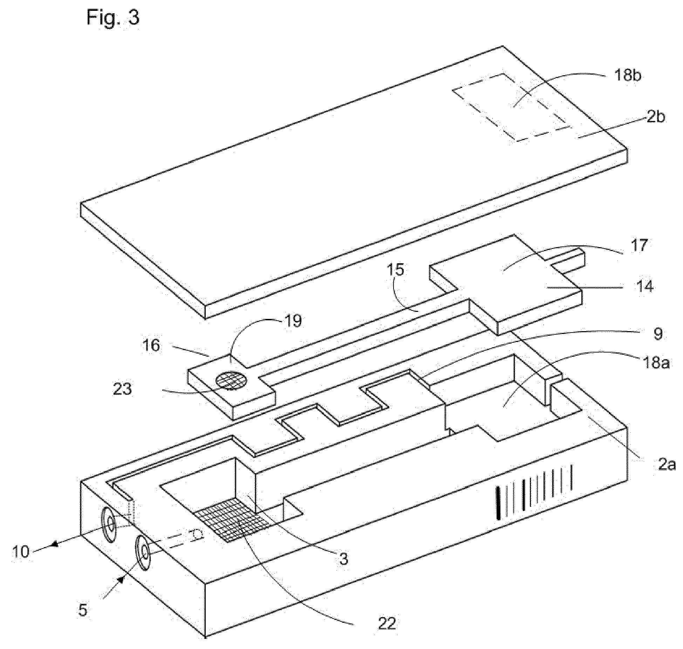

Die Figur 3 zeigt eine dritte Ausführungsform einer erfindungsgemäßen Kartusche 1, die im Wesentlichen denselben Aufbau wie die in den Figuren 1 und 2 dargestellten Kartuschen besitzt.

Die Kartusche 1 umfasst ein längliches Gehäuse 2 mit einem Gehäusekörper 2a und einem Deckel 2b, die jeweils eine Länge von 50 mm besitzen. Die Länge des Gehäuses kann zwischen 20 mm und 80 mm liegen. In dem Gehäuse 2 ist ein Probenraum 3 zum Aufnehmen eines Probenfluids ausgebildet. An einer Randfläche des Probenraums 3 ist ein Reagenz als in dem Probenfluid lösliche Beschichtung 22 vorgesehen.

Der Gehäusekörper 2a weist einen Einfüllkanal 4 auf, der sich ausgehend von einer in einer Wand des Gehäusekörpers 2a vorgesehenen Einfüllöffnung 5 in den Probenraum 3 erstreckt. Dem Einfüllkanal 4 ist ein Durchstechventil 6 als Einfüllkanalverschlusselement zugeordnet. Das Durchstechventil 6 ist derart ausgebildet, dass es eine Nadel 7 einer medizinischen Spritze 8 dicht umschließt, die in den Einfüllkanal 4 eingeführt ist.

Weiterhin weist der Gehäusekörper 2a einen Überlaufkanal 9 auf, der sich ausgehend von einer in einer Wand des Gehäusekörpers 2a vorgesehenen Überlauföffnung 10 in den Probenraum 3 erstreckt. Dem Überlaufkanal 9 ist eine hydrophobe und/oder hydrophile Fritte 11 als Überlaufkanalverschlusselement zugeordnet, die derart ausgebildet ist, dass durch sie Luft, nicht aber Probenfluid aus dem Probenraum 3 entweichen kann. Alternativ zu der hydrophoben und/oder hydrophilen Fritte 11 kann dem Überlaufkanal 9 auch eine gasdurchlässige Membran zugeordnet sein. Der Einfüllkanal 4 und der Überlaufkanal 10 münden in gegenüberliegende Bereiche des Probenraums 3, so dass Probenfluid erst dann in den Überlaufkanal 9 gelangt, wenn der Probenraum 3 vollständig mit dem Probenfluid gefüllt ist. Der Überlaufkanal 9 weist einen mäanderförmigen Kontrollabschnitt 12 auf. Dadurch wird eine große Länge des Überlaufkanals 9 erreicht, so dass Probenfluid nicht sofort zu der Überlauföffnung 10 gelangt, wenn der Probenraum 3 vollständig gefüllt ist und weiter Probenfluid zugeführt wird. Im Bereich des mäanderförmigen Kontrollabschnitts 12 des Überlaufkanals 9 weisen der Deckel 2b und der Gehäusekörper 2a transparente Abschnitte derart auf, dass der Kontrollabschnitt 12 von außen optisch zugänglich ist.

An einer Außenfläche des Gehäusekörpers 2a, die gegenüberliegend zu der Einfüllöffnung 5 und der Überlauföffnung 10 angeordnet ist, ist ein Barcode 13 als Identifikationseinrichtung vorgesehen. Alternativ oder zusätzlich kann die Identifikationseinrichtung auch als RFID-Chip ausgebildet sein.

Die Kartusche 1 umfasst ferner ein Schwingelement 14, das einen mit dem Gehäuse 2 verbundenen und von diesem in den Probenraum 3 vorstehenden länglichen, in einer Schwingrichtung elastisch biegbaren Schwingabschnitt 15 aufweist. Der Schwingabschnitt 15 des Schwingelements 14 besitzt eine stabförmige Gestalt und weist quer zu seiner Längsrichtung eine rechteckige Querschnittskontur auf. Die Querschnittskontur weist quer zu der Längsrichtung und quer zu der Schwingrichtung eine Breite auf, die zu der maximalen Breite des Probenkontaktabschnitts in einem Verhältnis von ungefähr 0,2 steht, was mit einer geringen Dämpfung durch das Probenfluid einhergeht und zu einer entsprechend hohen Güte des Schwingelements führt.

An dem freien Ende des Schwingabschnitts 15 ist ein plattenförmiger Probenkontaktabschnitt 16 ausgebildet, der eine rechteckige Gestalt besitzt und an seinen gegenüberliegenden Hauptflächen zwei Haftflächen definiert. Seine quer zu der Schwingrichtung und quer zu der Längsrichtung gemessene Breite ist ungefähr doppelt so groß wie seine in der Längsrichtung gemessene Länge. Gegenüberliegend zu dem Probenkontaktabschnitt 16 ist an dem Schwingabschnitt 15 ein Befestigungsabschnitt 17 ausgebildet. Der Befestigungsabschnitt 17 ist plattenförmig ausgebildet und besitzt eine rechteckige Gestalt. Der Schwingabschnitt 15, der Probenkontaktabschnitt 16 sowie der Befestigungsabschnitt 17 des Schwingelements 14 sind einteilig aus Kunststoff hergestellt. Der Probenkontaktabschnitt 16 weist magnetisches Material in Form eines Permanentmagneten 23 auf. An Haftfläche des Probenkontaktabschnitts 16 ist eine Haftbeschichtung 19 aus Kunststoff ausgebildet, die bezogen auf das Probenfluid bessere Adhäsionseigenschaften als der Kunststoff des Probenkontaktabschnitts 16 aufweist. Die Eigenfrequenz des von dem ungeronnenen Probenfluid umgebenen Schwingelements 14 liegt bei ungefähr 38 Hz. Die Güte beträgt ca. 20.

Zur Befestigung des Schwingelements 14 ist in dem Gehäuse 2 eine Halterung 18 ausgebildet, in welcher der Befestigungsabschnitt 17 aufgenommen und positioniert ist. Die Halterung 18 weist eine zu dem Befestigungsabschnitt 17 korrespondierende Aufnahme 18a auf, die in dem Gehäusekörper 2a ausgebildet ist und in welcher der Befestigungsabschnitt 17 des Schwingelements 14 aufgenommen und positioniert ist. Ferner weist die Halterung 18 einen Klemmvorsprung 18b auf, der an dem Deckel 2b vorgesehen ist und in Richtung der Aufnahme 18a vorsteht, so dass der Befestigungsabschnitt 17 zwischen einem Boden der Aufnahme 18a und dem Klemmelement 18b eingeklemmt ist, wenn der Deckel 2b den Probenraum 3 und die Aufnahme 18a verschließt.

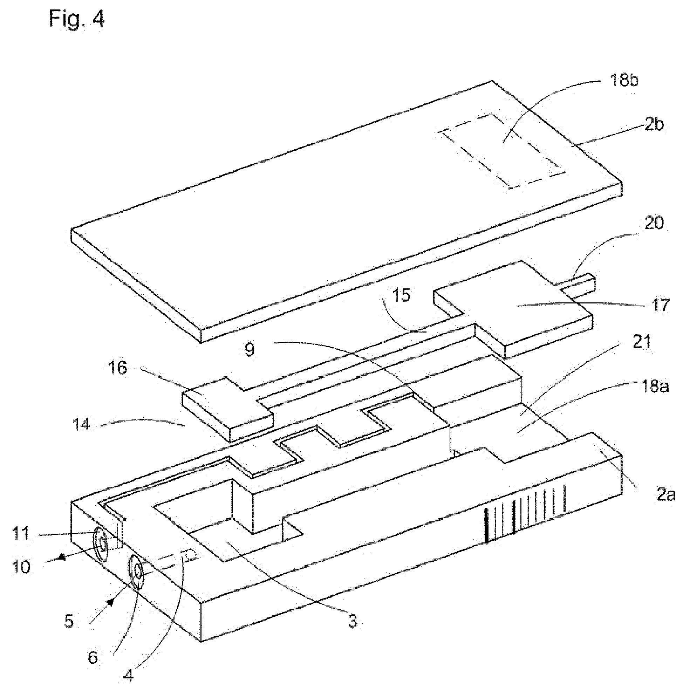

Die Figur 4 zeigt eine vierte Ausführungsform einer erfindungsgemäßen Kartusche 49, die im Wesentlichen denselben Aufbau wie die zuvor beschriebenen Kartuschen besitzt. Jedoch weist der Überlaufkanal 9 keinen mäanderförmigen Kontrollabschnitt auf. Zudem ist die Aufnahme 18a gänzlich als Durchlass 21 ausgebildet.

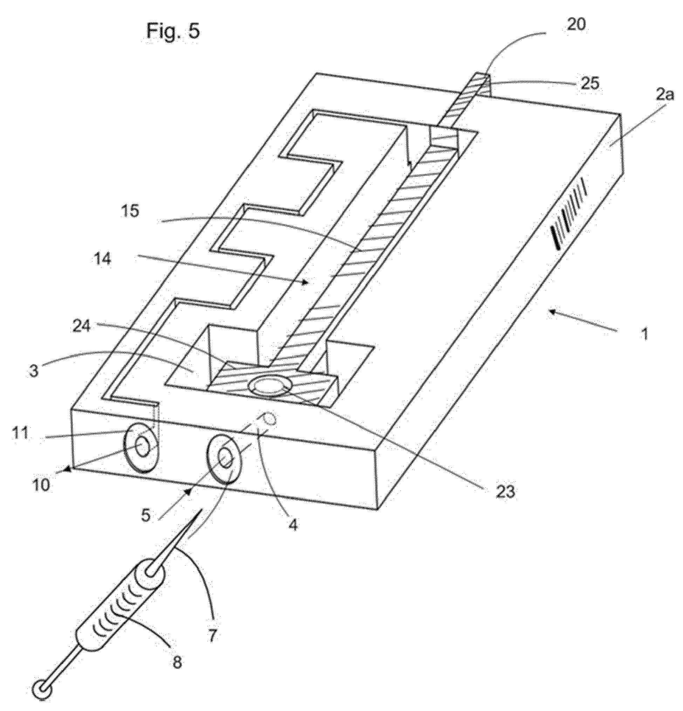

Die Figur 5 zeigt eine fünfte Ausführungsform einer erfindungsgemäßen Kartusche 1, bei der das Schwingelement 14 einteilig mit dem Gehäuse 2 ausgebildet ist.

Die Kartusche 1 umfasst ein längliches Gehäuse 2 mit einem Gehäusekörper 2a und einem Deckel (nicht dargestellt), die jeweils eine Länge von ungefähr 50 mm besitzen. Die Länge des Gehäuses kann zwischen 20 mm und 80 mm liegen. In dem Gehäuse 2 ist ein Probenraum 3 zum Aufnehmen eines Probenfluids ausgebildet.

Der Gehäusekörper 2a weist einen Einfüllkanal 4 auf, der sich ausgehend von einer in einer Wand des Gehäusekörpers 2a vorgesehenen Einfüllöffnung 5 in den Probenraum 3 erstreckt. Dem Einfüllkanal 4 ist ein Durchstechventil 6 als Einfüllkanalverschlusselement zugeordnet. Das Durchstechventil 6 ist derart ausgebildet, dass es eine Nadel 7 einer medizinischen Spritze 8 dicht umschließt, die in den Einfüllkanal 4 eingeführt ist.

Weiterhin weist der Gehäusekörper 2a einen Überlaufkanal (nicht dargestellt) auf, der sich ausgehend von einer in einer Wand des Gehäusekörpers 2a vorgesehenen Überlauföffnung 10 in den Probenraum 3 erstreckt. Dem Überlaufkanal ist eine hydrophobe und/oder hydrophile Fritte 11 als Überlaufkanalverschlusselement zugeordnet, die derart ausgebildet ist, dass durch sie Luft, nicht aber Probenfluid aus dem Probenraum 3 entweichen kann. Alternativ zu der hydrophoben und/oder hydrophilen Fritte 11 kann dem Überlaufkanal auch eine gasdurchlässige Membran zugeordnet sein. Der Einfüllkanal 4 und der Überlaufkanal 10 münden in gegenüberliegende Bereiche des Probenraums 3, so dass Probenfluid erst dann in den Überlaufkanal gelangt, wenn der Probenraum 3 vollständig mit dem Probenfluid gefüllt ist.

Die Kartusche 1 umfasst ferner ein Schwingelement 14, das mit dem Gehäuse 2 verbunden ist und einen von diesem in den Probenraum 3 vorstehenden länglichen, in einer Schwingrichtung elastisch biegbaren Schwingabschnitt 15 aufweist. Der Schwingabschnitt 15 des Schwingelements 14 besitzt eine stabförmige Gestalt und weist quer zu seiner Längsrichtung eine rechteckige Querschnittskontur auf. Die Querschnittskontur weist quer zu der Längsrichtung und quer zu der Schwingrichtung eine Breite auf, die zu der maximalen Breite des Probenkontaktabschnitts in einem Verhältnis von ungefähr 0,2 steht, was mit einer geringen Dämpfung durch das Probenfluid einhergeht und zu einer entsprechend hohen Güte des Schwingelements führt.

An dem freien Ende des Schwingabschnitts 15 ist ein plattenförmiger Probenkontaktabschnitt 16 ausgebildet, der eine rechteckige Gestalt besitzt und an seinen gegenüberliegenden Hauptflächen zwei Haftflächen definiert. Seine quer zu der Schwingrichtung und quer zu der Längsrichtung gemessene Breite ist ungefähr doppelt so groß wie seine in der Längsrichtung gemessene Länge. Der Schwingabschnitt 15 und der Probenkontaktabschnitt 16 des Schwingelements 14 sind einteilig aus Kunststoff hergestellt. Dabei ist das Schwingelement 14 einteilig mit dem Gehäuse 2 ausgebildet, beispielsweise an dieses angegossen. Der Probenkontaktabschnitt 16 weist einen Permanentmagneten 23 als magnetisches Material auf. An dem Probenkontaktabschnitt 16 ist eine elektrisch leitende Kondensatorfläche 24 ausgebildet, die mit einem an der Außenseite des Gehäusekörpers 2a vorgesehenen Kontaktbereich, der gleichzeitig als Markierungsvorsprung 20 ausgebildet ist, durch eine elektrische Leitung 25 verbunden. Die Kondensatorfläche 24 sowie die elektrische Leitung 25 sind durch Bedampfen des Schwingelements 14 mit Metall hergestellt. Alternativ können aber auch andere Beschichtungsverfahren verwendet werden, um eine Kondensatorfläche 24 sowie eine elektrische Leitung 25 an dem Schwingelement 14 auszubilden. Die Eigenfrequenz des von dem ungeronnenen Probenfluid umgebenen Schwingelements 14 liegt bei ungefähr 38 Hz. Die Güte beträgt ca. 20.

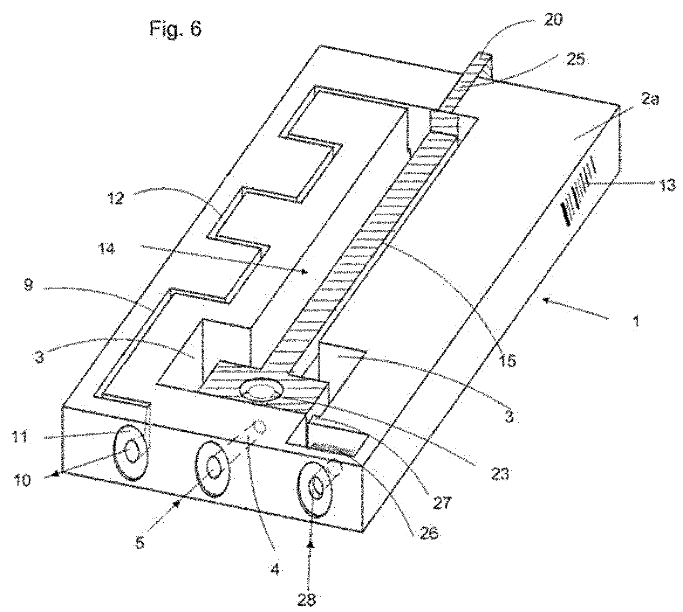

Die Figur 6 zeigt eine sechste Ausführungsform einer erfindungsgemäßen Kartusche 1, die im Wesentlichen denselben Aufbau wie die zuvor gezeigte Kartusche besitzt. Im Unterschied zu dieser umfasst sie eine Reagenzkammer 26, einen Mischkanal 27 sowie eine Mischöffnung 28.

In dem Gehäusekörper 2a der Kartusche 1 ist eine Reagenzkammer 26 ausgebildet, die ein Reagenz in Form einer mit dem Probenfluid mischbaren Flüssigkeit, bevorzugt in Form eines Lyophilisats enthält. Die Reagenzkammer 26 ist in einem Mischkanal 27 vorgesehen, der sich in einer Wand des Gehäusekörpers 2a ausgehend von einer Mischöffnung 28 in den Probenraum 3 erstreckt, so dass sich durch die Mischöffnung 28 eingefülltes Probenfluid mit dem Reagenz vermischt.

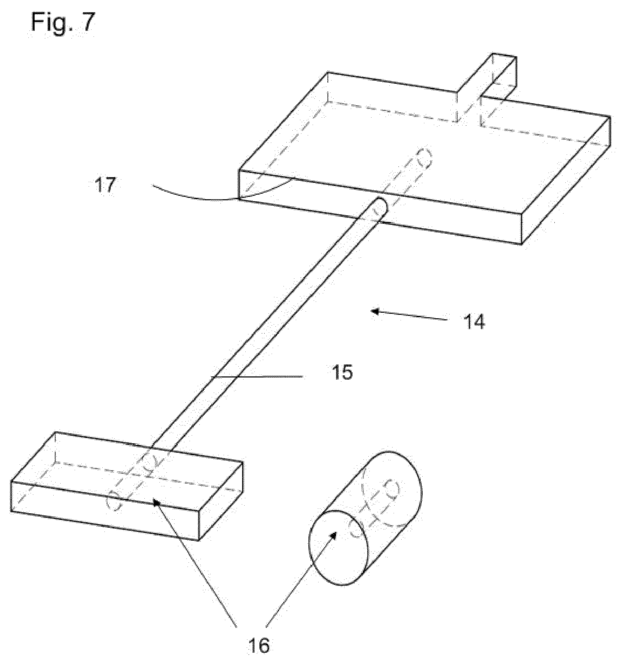

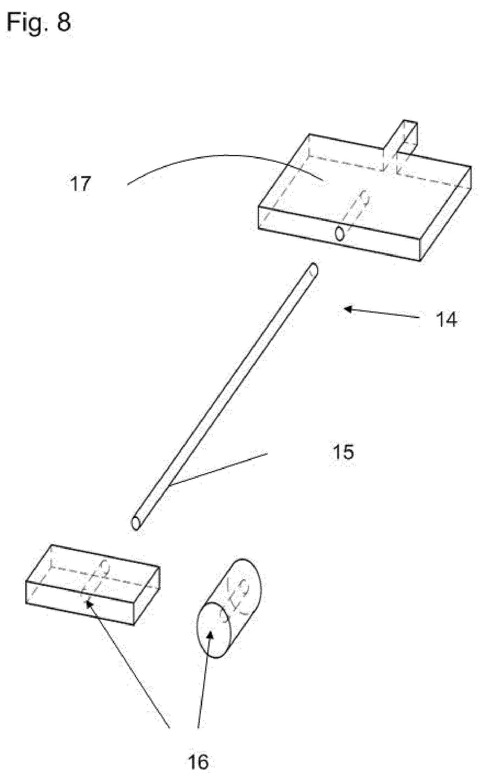

Die Figuren 7 und 8 zeigen eine siebte Ausführungsform einer erfindungsgemäßen Kartusche. Das Schwingelement 14 umfasst einen länglichen, in jede Schwingrichtung elastisch biegbaren Schwingabschnitt 15, der stabförmig ausgebildet ist und eine runde Querschnittskontur aufweist. An dem Schwingabschnitt 15 ist ein plattenförmiger Probenkontaktabschnitt 16 ausgebildet, der eine rechteckige Gestalt besitzt und an seinen gegenüberliegenden Hauptflächen zwei Haftflächen definiert. Seine quer zu der Schwingrichtung und quer zu der Längsrichtung gemessene Breite ist ungefähr doppelt so groß wie seine in der Längsrichtung gemessene Länge. Alternativ kann aber auch ein Probenkontaktabschnitt zylindrischer Gestalt vorgesehen sein, der an der Außenseite seines Zylindermantels eine Haftfläche definiert. Sein quer zu der Schwingrichtung und quer zu der Längsrichtung gemessener Zylinderdurchmesser ist ungefähr halb so groß wie seine in der Längsrichtung gemessene Zylinderlänge. Gegenüberliegend zu dem Probenkontaktabschnitt 16 ist ein plattenförmiger Befestigungsabschnitt 17 ausgebildet, der eine rechteckige Gestalt besitzt.

Das Schwingelement 14 ist in seiner Längsrichtung in mehrere Schwingelementabschnitte unterteilt, die durch eine Steckverbindung miteinander verbindbar sind. Die Schwingelementabschnitte umfassen den Schwingabschnitt 15, die alternativen Probenkontaktabschnitte 16 und den Befestigungsabschnitt 17. Dabei sind der Schwingabschnitt 15, die Probenkontaktabschnitte 16 und der Befestigungsabschnitt 17 aus unterschiedlichen Materialien hergestellt, können aber auch aus demselben Material hergestellt sein. Bei dieser Ausführungsform kann das Schwingelement 14 flexibel kombiniert werden. Beispielsweise kann statt des plattenförmigen Probenkontaktabschnitts 16 ein zylinderförmiger Probenkontaktabschnitt 16 auf den Schwingabschnitt 15 aufgesteckt sein. Auch hinsichtlich der weiteren Schwingelementabschnitte können Varianten vorgesehen sein. Die Eigenfrequenz des von dem ungeronnenen Probenfluid umgebenen Schwingelements 14 liegt bei ungefähr 38 Hz. Die Güte beträgt ca. 20.

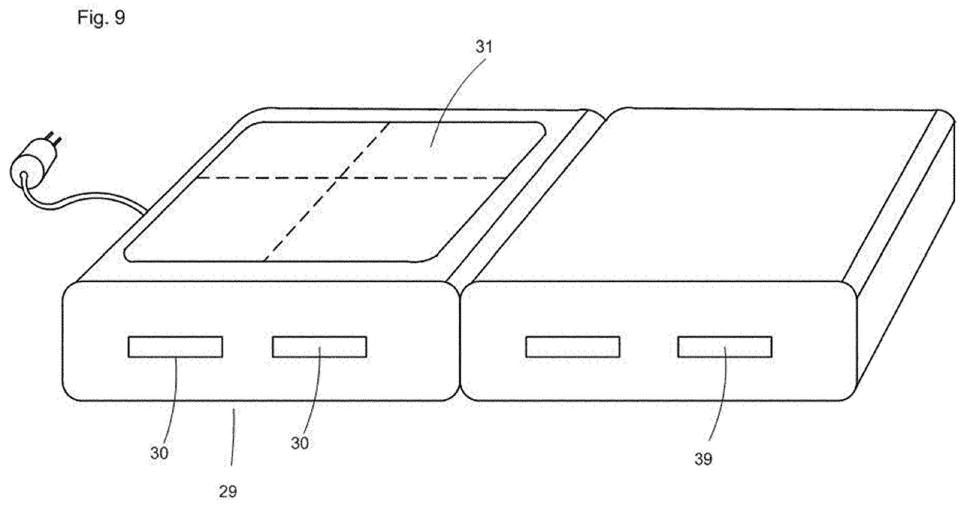

Die Figuren 9 bis 16 zeigen Vorrichtungen zur Koagulationsmessung gemäß zweier verschiedener Ausführungsformen der vorliegenden Erfindung.

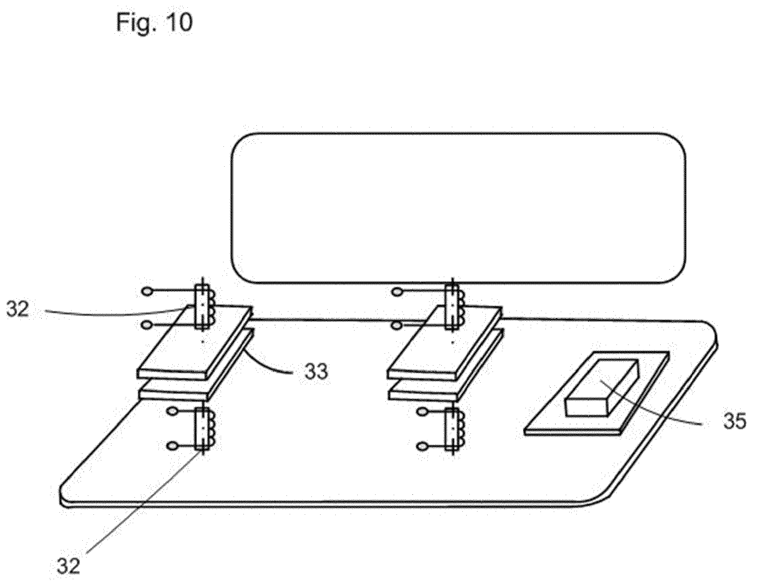

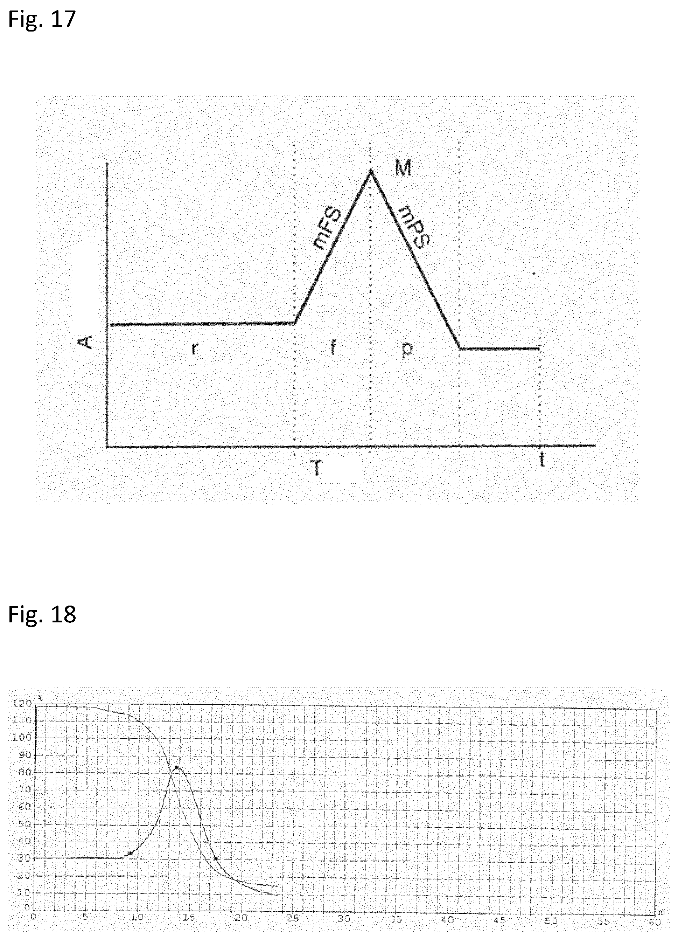

Die Figuren 9 und 10 zeigen eine Vorrichtung 29 zur Koagulationsmessung gemäß einer ersten Ausführungsform der vorliegenden Erfindung. Die Vorrichtung 29 umfasst zwei Kartuschenaufnahmen 30 zum Einsetzen einer erfindungsgemäßen Kartusche 1. Mindestens zwei Kartuschenaufnahmen 30 sind erforderlich, um zu Kontrollzwecken gleichzeitig mit dem zu vermessenden Probenfluid ein Kontrollfluid vermessen zu können. Ferner umfasst die Vorrichtung 29 eine Anzeigeeinheit 31, die die gleichzeitige Darstellung von vier Messkurven erlaubt, wie Sie in den Figuren 17 bis 20 dargestellt sind. Die Anzeigeeinheit umfasst entsprechend vier Quadranten, die den Kartuschenaufnahmen 30 zugeordnet sind. In die Anzeigeeinheit 31 ist ferner eine Touchscreen-Bedieneinheit integriert.

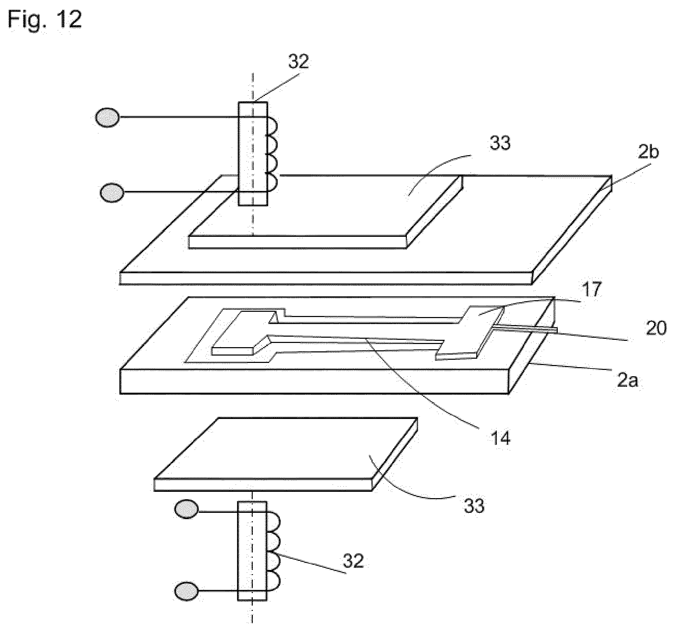

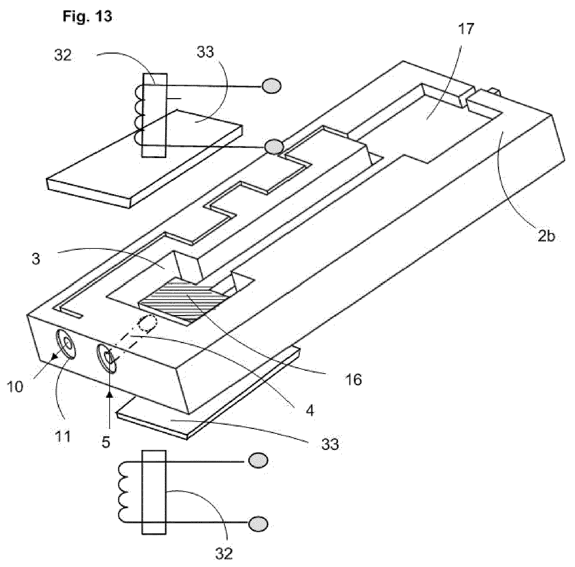

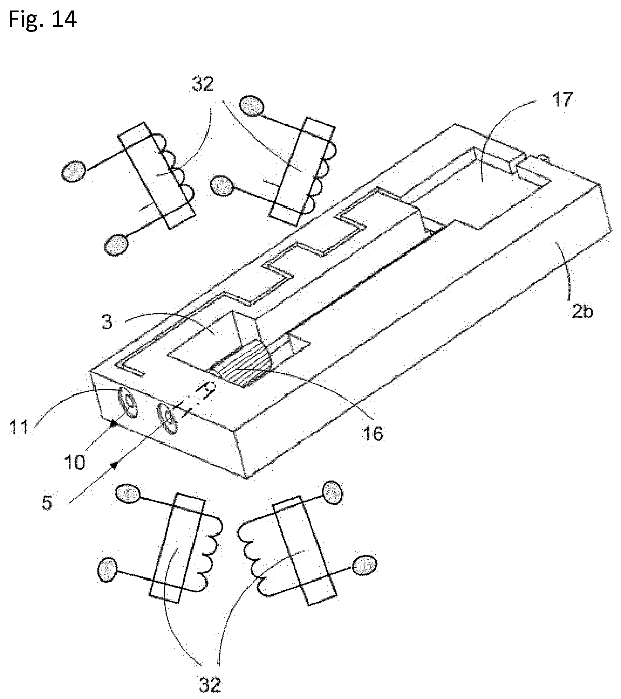

Weiterhin ist jeder Kartuschenaufnahme 30 eine Anregungseinheit aus zwei elektrisch erregbaren Magnetspulen 32 zugeordnet und relativ zu dieser derart angeordnet, dass sie den Probenkontaktabschnitt 16 eines Schwingelements 14 der eingesetzten Kartusche 1 wahlweise mit einer Magnetkraft beaufschlagen. Die Anregungseinheit 32 ist ausgelegt, um eine Anregungsfrequenz von 42 Hz zu erzeugen, die um ca. 4 Hz oberhalb der Eigenfrequenz des von ungeronnenem Probenfluid umgebenen Schwingelements 14 liegt. Infolge der Magnetkraft wird das Schwingelement 14 zu einer Schwingamplitude von ungefähr 1,5 mm angeregt, wobei die Schwingamplitude des Schwingelements 14 zwischen 0,5 mm und 2,5 mm variieren kann. Ferner ist jeder Kartuschenaufnahme 30 eine Sensoreinheit zugeordnet, die zwei Kondensatorplatten 33 umfasst, die sich parallel zu dem Probenkontaktabschnitt 16 des Schwingelements 14 der in die Kartuschenaufnahme 30 eingesetzten Kartusche 1 erstrecken und mit einer Kondensatorfläche 24 des Schwingelements 14 zwei Kondensatoren bilden, um die Position des Probenkontaktabschnitts 16 zu messen. Die Figuren 12 und 13 zeigen jeweils eine Anordnung einer in eine Kartuschenaufnahme 30 eingesetzten Kartusche 1 zwischen den der Kartuschenaufnahme 30 zugeordneten Magnetspulen 32 und Kondensatorplatten 33. Gemäß einer alternativen Variante können auch vier Magnetspulen 32 sowie vier Kondensatorplatten 33 in gleichen Winkelabständen um die Kartuschenaufnahme 30 angeordnet sein, um das Schwingelement der eingesetzten Kartusche zu einer zweidimensionalen Schwingung in Form einer Rührbewegung anzuregen und diese zu messen. Eine derartige Anordnung von vier Magnetspulen 32 ist in der Figur 14 schematisch angedeutet, die entsprechend eine Kartusche 1 mit einem Schwingelement 14 darstellt, dessen Schwingabschnitt 15 eine runde Querschnittskontur aufweist.

Eine alternative Sensoreinheit kann Reflexsensoren oder Hallsensoren umfassen, um die Position des Probenkontaktabschnitts 16 optisch oder magnetisch zu messen.

Jeder Kartuschenaufnahme 30 ist überdies eine Beheizungseinheit (nicht dargestellt) zugeordnet. Die Beheizungseinheit ist derart ausgebildet und angeordnet, dass das in einer eingesetzten Kartusche enthaltene Probenfluid eine Temperatur von ca. 37 °C erreicht und/oder beibehält. Jedoch kann die Beheizungseinheit auch abweichende Solltemperarturen des Probenfluids vorsehen.

Ferner ist jeder Kartuschenaufnahme 30 eine Füllkontrolleinheit 34 zugeordnet, die derart angeordnet ist, dass sie die Lichtdurchlässigkeit des Probenraums 3 oder des Kontrollabschnitts 12 messen kann.

Weiterhin ist jeder Kartuschenaufnahme 30 eine Erkennungseinheit (nicht dargestellt) zugeordnet, die derart ausgebildet und angeordnet ist, dass sie einen Markierungsvorsprung 20 einer eingesetzten Kartusche erkennt. Gleichzeitig weist die Erkennungseinheit einen elektrisch leitenden Kontaktbereich auf, der den Kontaktbereich der Kartusche 1 elektrisch leitend mit der Steuereinheit 35 derart verbindet, dass die Steuereinheit 35 die Kapazitäten der beiden aus den Kondensatorplatten 33 jeweils mit der Kondensatorfläche 24 gebildeten Kondensatoren während der Messung bestimmen kann.

Schließlich ist jeder Kartuschenaufnahme 30 eine Ableseeinheit (nicht dargestellt) zugeordnet, die ausgebildet und angeordnet ist, um eine an der eingesetzten Kartusche vorgesehene Identifikationseinrichtung 13 abzulesen.

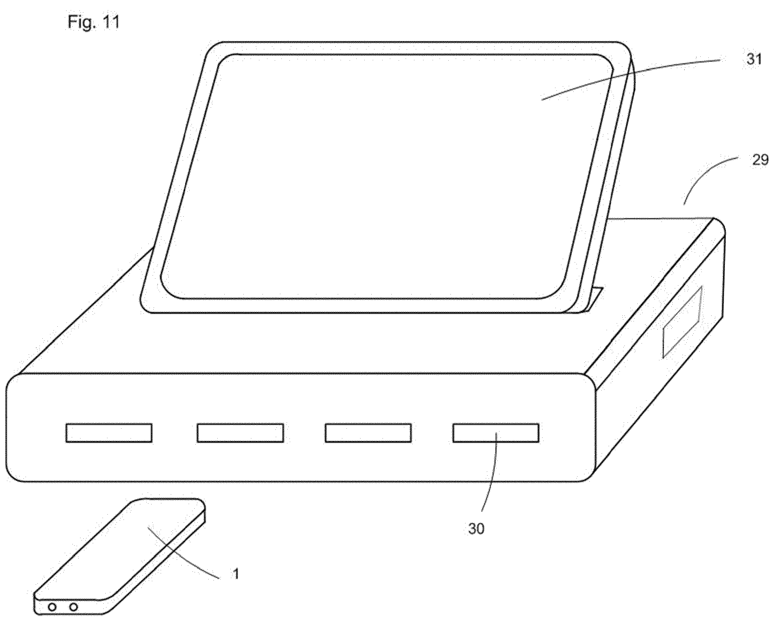

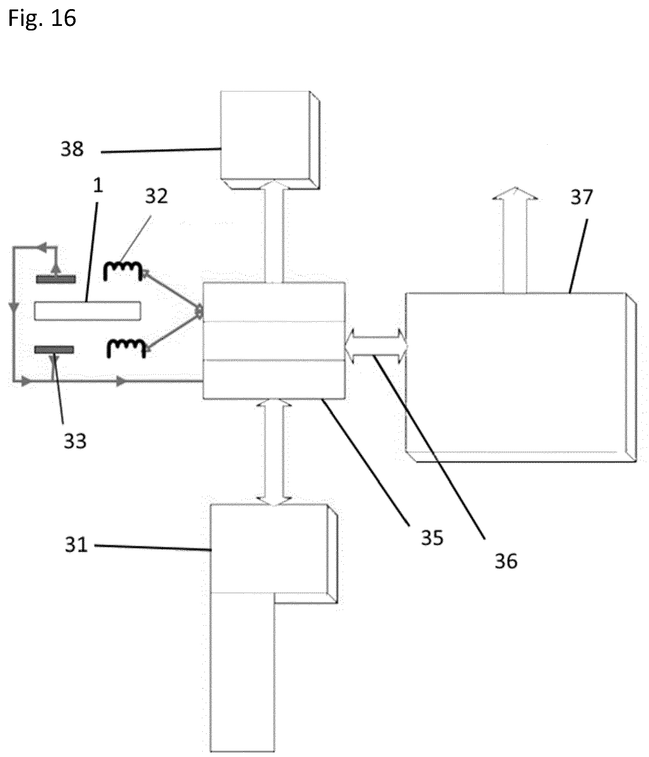

Die den Kartuschenaufnahmen 30 zugeordneten Magnetspulen 32, Kondensatorplatten 33, Beheizungseinheiten, Füllkontrolleinheiten 34, Ableseeinheiten und Erkennungseinheiten sind ebenso wie die Anzeigeeinheit 31 mit einer Steuereinheit 35 verbunden, die ausgebildet ist, um eine automatische Koagulationsmessung durchzuführen. Die Steuereinheit 35 umfasst eine Uhr (nicht dargestellt), um Datum und Uhrzeit des Starts einer Messung sowie die Messdauer seit dem Start der Messung zu ermitteln. Selbstverständlich ist die Steuereinheit 35 derart flexibel ausgelegt, dass Kartuschen gemäß unterschiedlicher Ausführungsformen der vorliegenden Erfindung verwendbar sind. Ein entsprechendes Blockschaltbild ohne Füllkontrolleinheiten, Ableseeinheiten, Uhr und Erkennungseinheiten ist in Figur 16 dargestellt, das neben den bereits beschriebenen Einheiten der Vorrichtung 29 eine Schnittstelleneinheit 36 zum Anschließen eines Personalcomputers (PC) 37 oder dergleichen sowie eines Druckers 38 oder dergleichen zeigt. Die Steuereinheit 35 ist für die Steuerung von bis zu vier Kartuschenaufnahmen 30 ausgelegt und erlaubt somit das Anschließen einer Erweiterungseinheit 39 mit zwei weiteren Kartuschenaufnahmen 30. Die Figur 11 zeigt eine Vorrichtung 29 zur Koagulationsmessung gemäß einer zweiten Ausführungsform der vorliegenden Erfindung mit vier Kartuschenaufnahmen 30.

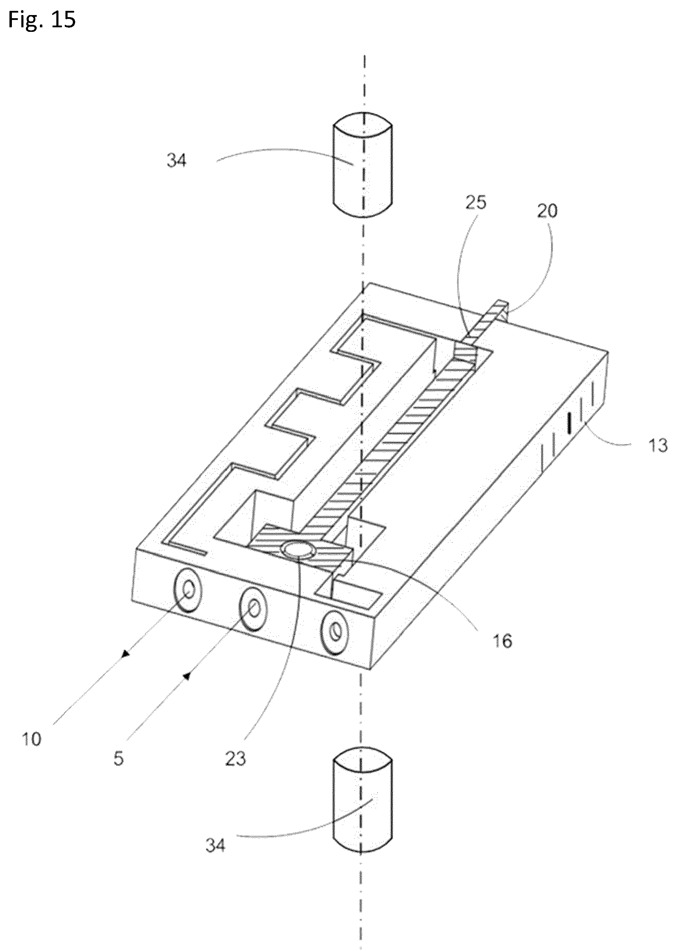

Zum Messen der Koagulation eines Probenfluids wird das Probefluid mittels einer Spritze 8 durch die Einfüllöffnung 5 oder die Mischöffnung 28 einer Kartusche 1 eingefüllt, bis der Probenraum 3 vollständig mit dem Probenfluid gefüllt ist. Die Befüllung lässt sich außerhalb der Kartuschenaufnahme 30 durchführen und deren Vollständigkeit mittels visueller Überprüfung des Kontrollabschnitts 12 feststellen. Alternativ kann man die Befüllung nach dem Einsetzen in eine Kartuschenaufnahme 30 vornehmen, wobei die Vollständigkeit durch die Füllkontrolleinheit 34 detektiert wird, die wahlweise die Lichtdurchlässigkeit des Probenraums 3 oder des Kontrollabschnitts 12 misst. Figur 15 zeigt eine Füllkontrolleinheit 34, die die Lichtdurchlässigkeit des Probenraums 3 misst.

Die Vorrichtung 29 erkennt eine eingesetzte Kartusche 1 an einem Markierungsvorsprung 20. Die Vorrichtung 29 startet automatisch den Messvorgang, wenn eine vollständig mit Probenfluid gefüllte Kartusche 1 in eine Kartuschenaufnahme 30 eingesetzt ist. Wenn die Identifikationseinrichtung 13 einer Kartusche Informationen über ihr Mindesthaltbarkeitsdatum vorsieht, kann die Vorrichtung 29 nach einem Vergleich mit dem aktuellen Messzeitpunkt einen Start der Messung ablehnen. Ferner kann die Identifikationseinrichtung 13 Angaben über die Herkunft des Probenfluids und ggf. enthaltene Reagenzien vorsehen, was eine präzise Zuordnung und Interpretation der ermittelten Messkurven erlaubt.

Während der Messung (Resonanzthrombelastographie) werden die Magnetspulen 32 mit elektrischem Wechselstrom einer Frequenz von ca. 42 Hz beaufschlagt, die um ca. 6 Hz oberhalb der Eigenfrequenz des Schwingelements in dem noch ungeronnenen Probenfluid liegt. Das von ihnen erzeugte magnetische Wechselfeld übt auf das magnetische Material des Probenkontaktabschnitts 17 des Schwingelements 14 der eingesetzten Kartusche eine zeitlich veränderliche Wechselkraft konstanter Amplitude aus, die das Schwingelement 14 zu periodischen Schwingungen in dem Probenfluid anregt. Während der fortschreitenden Gerinnung des Probenfluids erhöht sich allmählich die Eigenfrequenz des von diesem umgebenen Schwingelements 14. Entsprechend läuft die Schwingungsamplitude des Probenkontaktabschnitts 16 durch ein Maximum, wenn die Eigenfrequenz gleich der Anregungsfrequenz ist (Resonanz). Wenn sich der Probenkontaktabschnitt 16 zwischen den zugeordneten Kondensatorplatten 33 hin und her bewegt, verändern sich die Kapazitäten der beiden mit der Kondensatorfläche 24 gebildeten Kondensatoren gegenläufig. Dabei korrespondieren die Kapazitäten in jedem Zeitpunkt der Messung jeweils mit der Auslenkung des Probenkontaktabschnitts 16, so dass die Amplitude der Kapazitätsschwankung der Kondensatoren mit der Schwingungsamplitude des Schwingelements 14 korreliert. Selbstverständlich ist es ausreichend, dass die Sensoreinheit eine einzige Kondensatorplatte 33 umfasst. Bei der Messung ergeben sich die in den Figuren 17 bis 19 dargestellten Messkurven, bei denen für jeden Messzeitpunkt in der Abszisse die Zeit T und in der Ordinate die Schwingungsamplitude S abgetragen ist.

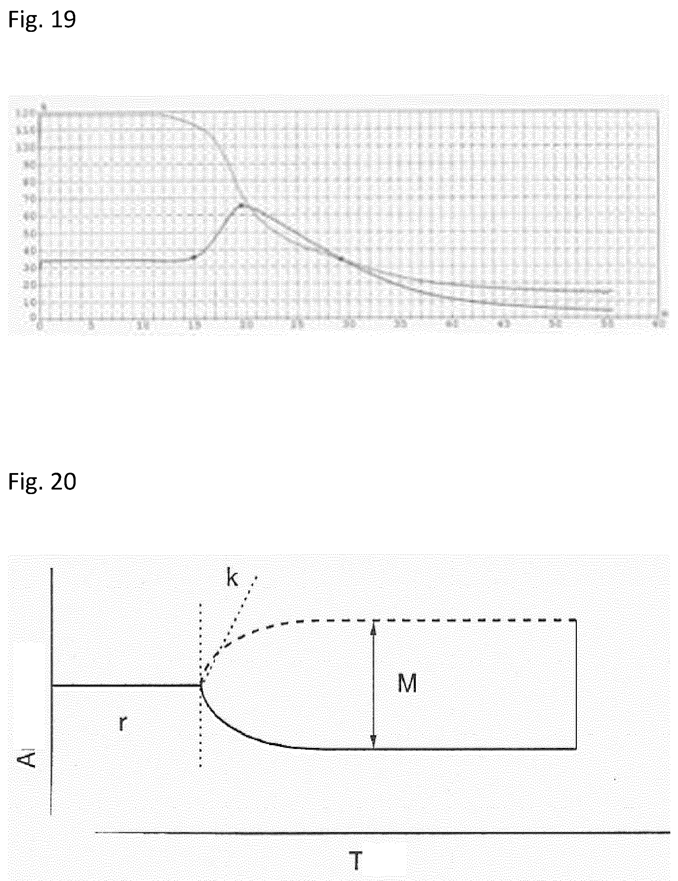

Bei einer alternativen Messung (Thrombelastographie, TEG) kann auch die Anregungsstärke gemessen werden, die erforderlich ist, um eine konstante Schwingungsamplitude aufrechtzuerhalten. Für die Anregung wird eine konstante Anregungsfrequenz leicht unterhalb der Eigenfrequenz des von dem ungeronnenem Probenfluid umgebenen Schwingelements 14 gewählt. Bei fortschreitender Koagulation des Probenfluids, d.h. zunehmender Festigkeit des Schwingelements 14, ist eine entsprechend zunehmende Anregungsstärke erforderlich, um die Schwingungsamplitude aufrechtzuerhalten. Wenn die erforderliche Anregungsstärke A über der Zeit T aufgetragen wird, ergibt sich bei ordnungsgemäßer Koagulation des Probenfluids ein Kurvenverlauf wie schematisch in Figur 20 dargestellt.

Ein wesentlicher Vorteil der erfindungsgemäßen Kartusche sowie der entsprechenden erfindungsgemäßen Vorrichtung 29 besteht darin, dass eine präzise Koagulationsmessung auch für ungeschultes Personal möglich ist. Die Integration eines Schwingelements 14 in einen abgeschlossenen Proberaum 3 einer Kartusche 1 erlaubt die einfache Herstellung kontrollierter Messbedingungen und erleichtert die Handhabung, was insbesondere für eine mobile Verwendung im Rahmen der Notrettung zuträglich ist. Zudem ist das in dem Proberaum 3 enthaltene Probefluid vor Umgebungseinflüssen geschützt.

### Bezugszeichenliste

- 1: Kartusche
- 2: Gehäuse
- 2a: Gehäusekörper
- 2b: Deckel
- 3: Probenraum
- 4: Einfüllkanal
- 5: Einfüllöffnung
- 6: Durchstechventil
- 7: Nadel
- 8: Spritze
- 9: Überlaufkanal
- 10: Überlauföffnung
- 11: Fritte
- 12: Kontrollabschnitt
- 13: Barcode
- 14: Schwingelement
- 15: Schwingabschnitt
- 16: Probenkontaktabschnitt
- 17: Befestigungsabschnitt
- 18: Halterung
- 18a: Aufnahme
- 18b: Klemmvorsprung
- 19: Haftbeschichtung
- 20: Markierungsvorsprung
- 21: Durchlass
- 22: Beschichtung
- 23: Permanentmagnet
- 24: Kondensatorfläche
- 25: Leitung
- 26: Reagenzkammer
- 27: Mischkanal
- 28: Mischöffnung
- 29: Vorrichtung
- 30: Kartuschenaufnahme
- 31: Anzeigeeinheit
- 32: Magnetspule
- 33: Kondensatorplatte
- 34: Füllkontrolleinheit
- 35: Steuereinheit
- 36: Schnittstelleneinheit
- 37: PC
- 38: Drucker
- 39: Erweiterungseinheit

## Patentansprüche

1. Kartusche (1) zum Einsetzen in eine Vorrichtung (29) zur Koagulationsmessung, mit einem Gehäuse (2), in dem ein Probenraum (3) zum Aufnehmen eines Probenfluids ausgebildet ist, und einem in dem Gehäuse (2) vorgesehen Schwingelement (14), das einen mit dem Gehäuse (2) verbundenen und von diesem in den Probenraum (3) vorstehenden länglichen, in wenigstens einer Schwingrichtung elastisch biegbaren Schwingabschnitt (15) und einen an dem freien Ende des Schwingabschnitts (15) angeordneten und wenigstens eine Haftfläche für das Probenfluid definierenden Probenkontaktabschnitt (16) aufweist, **dadurch gekennzeichnet, dass** der Schwingabschnitt (15) stabförmig mit einer insbesondere rechteckigen oder kreisförmigen Querschnittskontur ausgebildet ist, die quer zu der Längsrichtung des Schwingabschnitts (15) und quer zu der wenigstens einen Schwingrichtung des Schwingabschnitts (15) eine Breite aufweist, die zu einer maximalen Breite des Probenkontaktabschnitts in einem Verhältnis von höchstens 0,2, vorteilhaft höchstens 0,1 steht.

2. Kartusche nach Anspruch 1, **dadurch gekennzeichnet, dass** das von dem ungeronnenen Probenfluid umgebene Schwingelement (14) eine Eigenfrequenz, die in einem Bereich von 20 Hz bis 80 Hz, vorteilhaft zwischen 30 Hz und 50 Hz, vorteilhafter zwischen 35 Hz und 45 Hz liegt und bevorzugt 38 Hz beträgt, und/oder eine Güte besitzt, die in einem Bereich von 5 bis 45 liegt und bevorzugt wenigstens 20 beträgt.

3. Kartusche nach einem der Ansprüche 1 oder 2, **dadurch gekennzeichnet, dass** der Probenkontaktabschnitt (16) als eine insbesondere rechteckige Platte ausgebildet ist, deren Plattenlänge sich in der Längsrichtung des Schwingabschnitts (15) und deren Plattenbreite sich quer zu der Längsrichtung des Schwingabschnitts (15) und quer zu der wenigstens einen Schwingrichtung erstreckt, und die an gegenüberliegend angeordneten Hauptflächen zwei Haftflächen definiert, wobei das Verhältnis der Plattenbreite zu der Plattenlänge wenigstens 1, vorteilhaft wenigstens 2 beträgt und insbesondere an dem Probenkontaktabschnitt (16) eine elektrisch leitende Kondensatorfläche (24) ausgebildet ist, die mit einem an einer Außenseite des Gehäuses (2) vorgesehenen Kontaktbereich elektrisch leitend verbunden ist.

4. Kartusche nach einem Ansprüche 1 oder 2, **dadurch gekennzeichnet, dass** der Probenkontaktabschnitt (16) als ein Zylinder ausgebildet ist, dessen Zylinderachse sich in der Längsrichtung des Schwingabschnitts (15) erstreckt und der die Haftfläche an einer Außenseite des Zylindermantels definiert, wobei das Verhältnis der Zylinderlänge zu dem Zylinderdurchmesser wenigstens 1, vorteilhaft wenigstens 2 beträgt.

5. Kartusche nach einem der vorhergehenden Abschnitte, **dadurch gekennzeichnet, dass** der Probenkontaktabschnitt (16) aus einem magnetisierbaren oder magnetischen Material, insbesondere aus Stahl besteht oder aus Kunststoff hergestellt ist und einen Permanentmagneten (23) trägt, wobei insbesondere an jeder Haftfläche des Probenkontaktabschnitts (16) eine Haftbeschichtung (19) aus Kunststoff vorgesehen ist, die bezogen auf das Probenfluid bessere Adhäsionseigenschaften als das Material des Probenkontaktabschnitts (16) aufweist.

6. Kartusche nach einem der vorhergehenden Ansprüche, **dadurch gekennzeichnet, dass** an dem Gehäuse (2) eine Halterung (18) ausgebildet ist, in welcher ein insbesondere plattenförmiger und insbesondere rechteckiger Befestigungsabschnitt (17) des Schwingelements (14), der insbesondere gegenüberliegend zu dem Probenkontaktabschnitt (16) an dem Schwingabschnitt (15) des Schwingelements (14) ausgebildet ist, befestigbar oder befestigt ist, wobei insbesondere die Halterung (18) eine Aufnahme (18a), die korrespondierend zu dem Befestigungsabschnitt (17) des Schwingelements (14) ausgebildet ist und in welcher der Befestigungsabschnitt (17) des Schwingelements (14) aufgenommen und positioniert ist, und Klemmmittel (18b) umfasst, die den Befestigungsabschnitt (17) in der Aufnahme (18a) fixieren.

7. Kartusche nach Anspruch 6, **dadurch gekennzeichnet, dass** das Gehäuse (2) einen Gehäusekörper (2a), in dem der Probenraum (3) und die Aufnahme (18a) ausgebildet sind, und einen den Probenraum (3) und die Aufnahme (18a) verschließenden Deckel (2b) umfasst, an dem wenigstens ein Klemmelement (18b), insbesondere ein in Richtung der Aufnahme (18a) vorstehender Klemmvorsprung (18b) derart vorgesehen ist, dass der Befestigungsabschnitt (17) des Schwingelements (14) zwischen einem Boden der Aufnahme (18a) und dem Klemmelement (18b) eingeklemmt ist, wenn der Deckel (2b) den Gehäusekörper (2a) verschließt, wobei eine maximale Länge der Kartusche in dem Bereich von 20 mm bis 80 mm, vorteilhaft zwischen 40 mm und 60 mm liegt und bevorzugt 50 mm beträgt.

8. Kartusche nach einem der Ansprüche 6 oder 7, **dadurch gekennzeichnet, dass** das Schwingelement (14) in seiner Längsrichtung in mehrere Schwingelementabschnitte unterteilt ist, wobei die Schwingelementabschnitte insbesondere durch eine Steckverbindung miteinander verbindbar oder verbunden sind, wobei insbesondere die Schwingelementabschnitte den Schwingabschnitt (15), den Probenkontaktabschnitt (16) und/oder insbesondere den Befestigungsabschnitt (17) umfassen und/oder aus unterschiedlichen Materialien hergestellt sind.

9. Kartusche nach einem der vorhergehenden Ansprüche, **dadurch gekennzeichnet, dass** das Gehäuse (2) einen Einfüllkanal (4), der sich ausgehend von einer in einer Wand des Gehäuses (2) vorgesehenen Einfüllöffnung (5) in den Probenraum (3) erstreckt und dem ein Einfüllkanalverschlusselement (6), insbesondere ein Durchstechventil zugeordnet ist, und/oder einen Überlaufkanal (9) aufweist, der sich ausgehend von einer in einer Wand des Gehäuses (2) vorgesehenen Überlauföffnung (10) in den Probenraum (3) erstreckt und insbesondere einen insbesondere mäanderförmigen Kontrollabschnitt (12) aufweist, der durch einen transparenten Abschnitt des Gehäuses (2) von außen optisch zugänglich ist, und dem ein Überlaufkanalverschlusselement (11), insbesondere eine hydrophobe und/oder hydrophile Fritte oder eine gasdurchlässige Membran zugeordnet ist, wobei insbesondere der Einfüllkanal (4) und der Überlaufkanal (9) in gegenüberliegende Bereiche des Probenraums (3) münden.

10. Kartusche gemäß einem der vorhergehenden Ansprüche, **dadurch gekennzeichnet, dass** an einer Außenseite des Gehäuses (2) eine Markierung (20), die insbesondere als Markierungsvorsprung (20) ausgebildet ist, der von dem Gehäuse (2) auswärts vorsteht, wobei insbesondere der Markierungsvorsprung (20) an dem Befestigungsabschnitt (17) des Schwingelements (14) insbesondere gegenüberliegend zu dem Schwingabschnitt (15) ausgebildet ist und einen in dem Gehäuse (2) vorgesehenen Durchlass (21) durchsetzt, und wobei insbesondere der Markierungsvorsprung (20) des Schwingelements (14) gleichzeitig den außenseitigen Kontaktbereich der an dem Probenkontaktabschnitt (16) vorgesehenen Kondensatorfläche (24) bildet, und/oder an einer Außenfläche des Gehäuses (2) wenigstens eine Identifikationseinrichtung (13) vorgesehen ist, die insbesondere als Barcode oder als RFID-Chip ausgestaltet ist.

11. Kartusche nach einem der vorhergehenden Ansprüche, **dadurch gekennzeichnet, dass** innerhalb der Kartusche (1) ein Reagenz vorgesehen ist, das insbesondere als eine an dem Probenkontaktabschnitt (16) des Schwingelements (14) und/oder an einer Randfläche des Probenraums (3) vorgesehene und in dem Probenfluid lösliche Beschichtung (23) und/oder als eine mit dem Probenfluid mischbare Flüssigkeit (23), die in einer in dem Gehäuse (2) ausgebildeten und mit dem Probenraum (3) verbundenen Reagenzkammer (26) enthalten ist, vorgesehen ist.

12. Vorrichtung (29) zur Koagulationsmessung mittels einer Kartusche (1) nach einem der vorhergehenden Ansprüche, mit wenigstens einer Kartuschenaufnahme (30), in welche die Kartusche (1) einsetzbar ist, einer Anregungseinheit (32), die ausgebildet und angeordnet ist, um das Schwingelement (14) einer in die Kartuschenaufnahme (30) eingesetzten Kartusche (1) zum Schwingen mit einer Schwingamplitude anzuregen, einer Sensoreinheit (33), die Sensoreinheit (33) ausgebildet und angeordnet ist, um die Schwingamplitude des angeregten Schwingelements (14) zu messen, wobei die Anregungseinheit (32) und die Sensoreinheit (33) der wenigstens einen Kartuschenaufnahme (30) zugeordnet und zur automatischen Durchführung einer Koagulationsmessung mit einer Steuereinheit (35) verbunden sind, **dadurch gekennzeichnet, dass** die Anregungseinheit (32) ausgelegt ist, um Anregungsfrequenzen oberhalb der Eigenfrequenz des Schwingelements derart zu erzeugen, dass die Differenz zwischen der Anregungsfrequenz und der Eigenfrequenz in einem Bereich von 1 Hz bis 10 Hz, vorteilhaft zwischen 2 Hz und 6 Hz liegt und bevorzugt 4 Hz beträgt, und Schwingamplituden des Schwingelements (14) in einem Bereich von 0,5 mm bis 2,5 mm, vorteilhaft zwischen 1 mm und 2 mm und bevorzugt von 1,5 mm hervorzurufen.

13. Vorrichtung nach Anspruch 12, **dadurch gekennzeichnet, dass** die Anregungseinheit (32) wenigstens zwei elektrisch erregbare Magnetspulen (32) umfasst, die benachbart zu der wenigstens einen Kartuschenaufnahme (30) derart ausgebildet und angeordnet sind, dass sie den Probenkontaktabschnitt (16) des Schwingelements (14) der eingesetzten Kartusche (1) wahlweise mit einer Magnetkraft beaufschlagen, wobei insbesondere die Anregungseinheit (32) genau zwei Magnetspulen (32) umfasst, die auf gegenüberliegenden Seiten der Kartuschenaufnahme (30) angeordnet sind, oder die Anregungseinheit (32) genau vier Magnetspulen (32) umfasst, die in gleichen Winkelabständen um die Kartuschenaufnahme (30) angeordnet sind und/oder die Sensoreinheit (33) wenigstens eine, insbesondere genau zwei Kondensatorplatten (33) umfasst, die sich parallel zu dem Probenkontaktabschnitt (16) des Schwingelements (14) der in die Kartuschenaufnahme (30) eingesetzten Kartusche (1) erstrecken.

14. Vorrichtung nach einem der Ansprüche 12 oder 13, **dadurch gekennzeichnet, dass** der wenigstens einen Kartuschenaufnahme (30) eine mit der Steuereinheit (35) verbundene Beheizungseinheit, die derart ausgebildet und angeordnet ist, dass das in einer eingesetzten Kartusche (1) enthaltene Probenfluid eine bestimmte Temperatur erreicht und/oder beibehält, und/oder eine mit der Steuereinheit (35) verbundene Füllkontrolleinheit (34) zugeordnet ist, die derart ausgebildet und angeordnet ist, dass sie die Füllung der Kartusche (1) insbesondere optisch erkennt, und/oder eine mit der Steuereinheit (35) verbundene Erkennungseinheit zugeordnet ist, die derart ausgebildet und angeordnet ist, dass sie eine Markierung (20) einer eingesetzten Kartusche (1) erkennt, und/oder wenigstens eine mit der Steuereinheit (35) verbundene Ableseeinheit zugeordnet ist, die ausgebildet und angeordnet ist, um eine an der eingesetzten Kartusche (1) vorgesehene Identifikationseinrichtung (13) abzulesen.

15. Vorrichtung nach einem der Ansprüche 12 bis 14, **dadurch gekennzeichnet, dass** eine Mehrzahl von Kartuschenaufnahmen (30), insbesondere zwei Kartuschenaufnahmen (30) vorgesehen sind, wobei die Vorrichtung (29) insbesondere um weitere, insbesondere zwei Kartuschenaufnahmen (30) erweiterbar ist.
